(19) **Europäisches Patentamt / European Patent Office / Office européen des brevets**

(11) **EP 2 197 513 B1**

(12) **EUROPEAN PATENT SPECIFICATION**

(45) Date of publication and mention
of the grant of the patent:
**26.04.2017 Bulletin 2017/17**

(51) Int Cl.:
***A61M 1/10*** (2006.01)

(21) Application number: **08838971.3**

(86) International application number:
**PCT/US2008/011663**

(22) Date of filing: **10.10.2008**

(87) International publication number:
**WO 2009/051669 (23.04.2009 Gazette 2009/17)**

(54) **Blood circuit assembly for a dialysis unit**

Blutkreislaufanordnung für eine Dialyseeinheit

Ensemble de circuit sanguin pour une unité de dialyse

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **12.10.2007 US 871793**
**12.10.2007 US 871787**
**12.10.2007 US 871680**
**12.10.2007 US 871712**
**27.02.2008 US 38648**
**27.02.2008 US 38474**
**27.08.2008 US 199196**
**27.08.2008 US 198947**
**27.08.2008 US 199077**
**27.08.2008 US 199068**
**27.08.2008 US 199055**
**27.08.2008 US 199062**
**27.08.2008 US 199166**
**27.08.2008 US 199176**

(43) Date of publication of application:
**23.06.2010 Bulletin 2010/25**

(60) Divisional application:
**11150584.8 / 2 319 551**
**17159187.8**
**16152813.8 / 3 037 117**

(73) Proprietor: **DEKA Products Limited Partnership Manchester, NH 03101 (US)**

(72) Inventors:
• **BODWELL, Jesse, T.**
**Manchester, New Hampshire 03109 (US)**
• **CHAWAN, Arun, D.**
**Manchester, New Hampshire 03104 (US)**
• **COLLINS, David, E.**
**Manchester, New Hampshire 03104 (US)**
• **DEMERS, Jason, A.**
**Manchester, New Hampshire 03104 (US)**
• **PRESCOTT, Shannon**
**Loudon, New Hampshire 03307 (US)**
• **KAMEN, Dean**
**Bedford, New Hampshire 03110 (US)**
• **MARCEK, Geoffrey, A.**
**Houston Texas 77006 (US)**
• **GRANT, Kevin, L.**
**Litchfield, New Hampshire 03052 (US)**
• **WILT, Michael, J.**
**Windham, New Hampshire 03087 (US)**
• **DALE, James, D.**
**Nashua, New Hampshire 03062 (US)**

(74) Representative: **Hoffmann Eitle**
**Patent- und Rechtsanwälte PartmbB**
**Arabellastraße 30**
**81925 München (DE)**

(56) References cited:
**WO-A-99/10028          WO-A-2006/120415**
**WO-A-2008/106191     WO-A1-2008/053259**
**US-A- 4 299 784          US-A- 5 947 931**
**US-A1- 2005 069 425   US-A1- 2007 253 463**

## Description

## FIELD OF INVENTION

The present invention generally relates to hemodialysis and similar dialysis systems, e.g., systems able to treat blood or other bodily fluids extracorporeally.

## BACKGROUND

Many factors make hemodialysis inefficient, difficult, and expensive. These factors include the complexity of hemodialysis, the safety concerns related to hemodialysis, and the very large amount of dialysate needed for hemodialysis. Moreover, hemodialysis is typically performed in a dialysis center requiring skilled technicians. Therefore any increase in the ease and efficiency of the dialysis process could have an impact on treatment cost or patient outcome.

WO 2006/120415 discloses a dialysis machine which uses a disposable cartridge having a blood flowpath for carrying a volume of blood to be treated in a dialyser and a dialysate flowpath, isolated from the blood flowpath, for delivery of dialysate solution through the dialyser. The cartridge is slotted, horizontally into an engine section of the machine between first and second platens which close to retain the cartridge, when inserted.

US 2007/0253463 A1 discloses thermal and conductivity sensing systems, devices and methods, for example in relation to a heat exchanger. This document does not disclose a blood circuit assembly for a dialysis unit, nor an organising tray having a pair of handles arranged for gripping by a user for mounting or dismounting the blood circuit assembly on a front panel of the dialysis unit, nor a pair of dialyzer connections arranged to connect to the inlet and outlet of a dialyzer.

WO 2008/053259 A1 discloses a hemo(dia)filtration apparatus.

According to a first aspect of the present invention, there is provided a blood circuit assembly for a dialysis unit, comprising: an organizing tray having a pair of handles arranged for gripping by a user for installing the blood circuit assembly on a dialysis unit; a pair of pneumatic pumps mounted to the organizing tray for circulating blood received from a patient through a circuit including a dialyzer unit and returned to the patient; an air trap mounted to the organizing tray arranged to remove air from blood circulating in the circuit; a pair of dialyzer connections arranged to connect to the inlet and outlet of a dialyzer unit, and a pair of blood line connectors, one inlet blood line connector for receiving blood from the patient and providing blood to the pneumatic pumps and the other outlet blood line connector for returning blood to the patient.

Advantageously, embodiments of the invention may provide a blood circuit assembly for a dialysis unit arranged to allow the replacement of most or all blood circuit components in a single operation.

In one embodiment, an anticoagulant connection is provided for engaging with an anticoagulant source and providing anticoagulant into the blood circuit. For example, the anticoagulant connection may include a pump for pumping anticoagulant from the anticoagulant source, such as heparin from a vial of heparin, to the circuit. The anticoagulant connection may include a vial holder arranged to hold two or more differently sized vials, and a spike to pierce the vial. In one embodiment, the pair of pneumatic pumps, the anticoagulant connection, and the anticoagulant pump are part of a pump cassette.

The organizing tray may also include openings adjacent each of the handles for receiving retaining tabs on a dialysis unit that engage with the blood circuit assembly and retain the blood circuit assembly on the dialysis unit.

In one embodiment, the inlet blood line connector is connected to an inlet for the pump cassette, an outlet for the pump cassette is connected to a dialyzer inlet connector, a dialyzer outlet connector is connected to an inlet of the air trap, and an outlet of the air trap is connected to the outlet blood line connector. The inlet of the air trap may be located above the outlet of the air trap when the blood circuit assembly is mounted to a dialysis unit, e.g., to aid in trapping of air circulating in the circuit during treatment. The blood line connectors may be arranged for a threaded luer-type connection to a patient access, as well as be arranged for a press-in type connection to the dialysis unit. Such an arrangement may make it easier for an operator to connect the blood line connectors to the dialysis unit after treatment (e.g., for later disinfection and/or priming of the blood circuit) while allowing the connectors to engage with standard luer-type connectors at a patient blood access.

In one embodiment, the organizing tray may include circuit tube engagement members having a hole or slot through which a respective circuit tube passes. The engagement members may engage with the respective circuit tube to allow the circuit tube to be pulled and stretched for engagement with an occluder of the dialysis unit. For example, the circuit tubes of the blood circuit assembly may include silicone tubing that has to be stretched (and thereby reduced in diameter) to engage with an occluder. The circuit tube engagement members may resist the pull of an operator on the tubes, allowing the tubes to be stretched and placed in engagement with the occluder.

According to a second aspect of the invention, there is provided a method for replacing a blood circuit assembly according to the first aspect of a dialysis unit, the method including grasping the pair of handles on the organizing tray that is mounted to a dialysis unit, disengaging locking tabs of the dialysis unit from the blood circuit assembly to free the blood circuit assembly from the dialysis unit, and pulling on the handles on the organizing tray of the blood circuit assembly to remove the blood circuit assembly from the dialysis unit. Disengagement of the locking tabs may be performed by flexing the locking tabs away from each other such that each locking tab is moved toward a nearest one of the handles. After removal of the blood circuit assembly, a replacement blood circuit assembly may be provided, openings in the organizing tray of the replacement blood circuit assembly may be aligned with the locking tabs so that each locking tab is received into a respective opening, and the organizing tray may be pushed relative to the dialysis unit such that the locking tabs engage with the replacement blood circuit assembly to mount the replacement blood circuit assembly to the dialysis unit. Mounting the replacement blood circuit assembly may also involve connecting control ports on the dialysis unit to mating ports on the assembly so that fluid control signals may be provided for pumps and valves of the blood circuit assembly. Other blood circuit connections may be made, such as inlet and outlet connections for the dialyzer, and the blood line connectors may be connected to receive dialysate into the blood circuit

Other advantages and novel features of the present invention will become apparent from the following detailed description of various non-limiting embodiments of the invention when considered in conjunction with the accompanying figures.

**BRIEF DESCRIPTION OF THE DRAWINGS**

Aspects of the invention are described with reference to illustrative embodiments, which are described, by way of example only, with reference to the drawings in which like numerals reference like elements, and wherein:

FIG. 1 is a schematic representation of fluid handling components of a hemodialysis system;
FIG. 2 shows a schematic fluid flow diagram for the dialysis system of FIG. 1;
FIG. 3 is a schematic fluid flow diagram for the blood flow circuit of FIG. 2;
FIG. 4 is a schematic fluid flow diagram for the balancing circuit of FIG. 2;
FIG. 5 is a schematic fluid flow diagram for the directing circuit of FIG. 2;
FIG. 6 is a schematic fluid flow diagram for the mixing circuit of FIG. 2;
FIG. 7 is a right front perspective view of a hemodialysis system;
FIG. 8 is a left rear perspective view of the hemodialysis system of FIG. 7;
FIG. 9 is a front view of the hemodialysis system of FIG. 7;
FIG. 10 is a right front perspective view of the view of the hemodialysis system of FIG. 7 with the doors in a first open position;
FIG. 11 is a top view of the hemodialysis system of FIG. 10;
FIG. 12 is a front view of the hemodialysis system of FIG. 10;

FIG. 13 is a right side view of the hemodialysis system of FIG. 10;
FIG. 14 is a right front perspective view of the view of the hemodialysis system of FIG. 7 with the doors in a second open position;
FIG. 15 is a top view of the hemodialysis system of FIG. 14;
FIG. 16 is a front view of the hemodialysis system of FIG. 14;
FIG. 17 is a front view of the hemodialysis system of FIG. 7 with the doors in an open position exposing a front panel of the system;
FIG. 18 is a front view of an embodiment of a blood circuit assembly according to the present invention and for use with the system of FIG. 7;
FIG. 19 right perspective view of a organizing tray for the blood circuit assembly of FIG. 18;
FIG. 20 is a left rear perspective view of the blood circuit assembly of FIG. 18;
FIG. 21 shows a left front perspective view of the front panel of the system of FIG. 7;
FIG. 22 shows a front view of the front panel of the system of FIG. 7;
FIG. 23 shows a front view of the front panel of the system of FIG. 7 with a pair of mounting features for the dialyzer;
FIG. 24 shows a side view of a dialyzer with quick-connect fittings attached to the dialysate inlet/outlet ports of the dialyzer;
FIG. 25 shows a right perspective view of a reagent supply for use with the system of FIG. 7;
FIG. 26 shows a perspective view of an E-prong connector for the reagent supply of FIG. 25 and a corresponding connection point at the front panel of the hemodialysis system;
FIG. 27 shows a perspective view of a pair of blood line connectors for the blood circuit assembly and a corresponding connection point at the front panel of the hemodialysis system; and
FIG. 28 shows a side view of a blood line connector and connection point of FIG. 27;
FIG. 29 is a perspective view of a pod pump;
FIG. 30 is a sectional view of a pod-pump that may be incorporated into fluid-control cassettes;
FIG. 31 is a sectional view of a valve that may be incorporated into fluid-control cassettes;
FIGS. 32A and 32B are top and section views of a pod pump with a laminated construction;
FIGS. 33A and 33B are top and section views of a pod pump with a laminated construction;
FIGS. 34A - 34C are exploded and section views of a pod pump cassette;
FIGS. 35A - 35B are pictorial views of a pod pump cassette;
FIGS. 36A - 36B show a pump cassette incorporating two pod pumps of the type shown in FIG. 30 and a number of valves of the type shown in FIG. 31 along with various fluid paths and other components;

FIG. 37 is a schematic illustration of a pumping system;

FIG. 38A is a flow chart illustrating a series of steps in a pumping cycle;

FIG. 38B is a flow chart illustrating a series of substeps of the pumping cycle of FIG. 38A for performing volume calculation and air detection;

FIG. 38C is a flow chart illustrating a series of substep of the pumping cycle of FIG. 38A for detecting the presence of a gas in a pump chamber;

FIG. 39 shows a balancing pod;

FIG. 40 shows a sensing probe coupled with a thermal well outside of a fluid line;

FIG. 41 is a perspective view of a tubing occluder;

FIG. 42 is a perspective view of a blood pump cassette;

FIG. 43 is a perspective view of the inside top plate of the blood pump cassette depicted in FIG. 42;

FIG. 44 is a perspective view of the mid-plate of the blood pump cassette depicted in FIG. 42.

## DETAILED DESCRIPTION

Embodiments described herein generally relate to hemodialysis and similar dialysis systems. Illustrative embodiments described herein involve, in some cases, interrelated products, alternative solutions to a particular problem, and/or a plurality of different uses of one or more systems and/or articles. Although the various systems and methods described herein are described in relation to hemodialysis, it should be understood that the various systems and method described herein are applicable to other dialysis systems and/or in any extracorporeal system able to treat blood or other bodily fluids, such as hemofiltration, hemodiafiltration, etc.

Embodiments described herein are generally directed to new systems for hemodialysis and the like, such as hemofiltration systems, hemodiafiltration systems, plasmapheresis systems, etc. Accordingly, although the various systems and methods described herein are described in relation to hemodialysis, it should be understood that the various systems and method described herein are applicable to other dialysis systems and/or in any extracorporeal system able to treat blood or other bodily fluids, such as plasma.

As discussed below, a hemodialysis system typically includes a blood flow path and a dialysate flow path. It should be noted that within such flow paths, the flow of fluid is not necessarily linear, and there may be any number of "branches" within the flow path that a fluid can flow from an inlet of the flow path to an outlet of the flow path. Examples of such branching are discussed in detail below. In the blood flow path, blood is drawn from a patient, and is passed through a dialyzer, before being returned to the patient. The blood is treated by the dialyzer, and waste molecules (e.g., urea, creatinine, etc.) and water are passed from the blood, through a semipermeable membrane in the dialyzer, into a dialysate solution that passes through the dialyzer by the dialysate flow path. In various examples, blood may be drawn from the patient from two lines (e.g., an arterial line and a venous line, i.e., "dual needle" flow), or in some cases, blood may be drawn from the patient and returned through the same or catheter needle (e.g., the two lines or lumens may both be present within the same needle, i.e., a form of "dual lumen" flow). In still other examples, a "Y" site or "T" site is used, where blood is drawn from the patient and returned to the patient through one patient connection having two branches (one being the fluid path for the drawn blood, the second the fluid path for the return blood, i.e., a form of "single needle" flow). The patient may be any subject in need of hemodialysis or similar treatments, including non-human subjects, such as dogs, cats, monkeys, and the like, as well as humans.

In the dialysate flow path, fresh dialysate is prepared and is passed through the dialyzer to treat the blood from the blood flow path. The dialysate may also be equalized for blood treatment within the dialyzer (i.e., the pressure between the dialysate and the blood are equalized), often exactly, or in some cases, at least within about 1% or about 2% of the pressure of the blood. In some cases, it may be desirable to maintain a greater pressure difference (either positive or negative) between the blood flow path and dialysate flow path. After passing through the dialyzer, the used dialysate, containing waste molecules (as discussed below), is discarded in some fashion. The dialysate in some cases may be re-circulated in a "multi-pass" arrangement, which may be beneficial in capturing larger molecules having low mobility across the dialyzer. In some cases, the dialysate is heated prior to treatment of the blood within the dialyzer using an appropriate heater, such as an electrical resistive heater. The dialysate may also be filtered to remove contaminants, infectious organisms, debris, and the like, for instance, using an ultrafilter. The ultrafilter may have a pore size chosen to prevent species such as these from passing therethrough. For instance, the pore size may be less than about 0.3 micrometers, less than about 0.2 micrometers, less than about 0.1 micrometers, or less than about 0.05 micrometers, etc. The dialysate is used to draw waste molecules (e.g., urea, creatinine, ions such as potassium, phosphate, etc.) and water from the blood into the dialysate through osmosis or convective transport, and dialysate solutions are well-known to those of ordinary skill in the art.

The dialysate typically contains various ions such as sodium, chloride, bicarbonate, potassium and calcium that are similar in concentration to that of normal blood. In some cases, the bicarbonate, may be at a concentration somewhat higher than found in normal blood. Typically, the dialysate is prepared by mixing water from a water supply with one or more ingredients: an "acid" (which may contain various species such as acetic acid, dextrose, NaCl, CaCl, KCl, MgCl, etc.), sodium bicarbonate (NaHCO3), and/or sodium chloride (NaCl). The preparation of dialysate, including using the appropriate concentrations of salts, osmolarity, pH, and the like, is well-known to those of ordinary skill in the art. As discussed in detail below, the dialysate need not be prepared at the same rate that the dialysate is used to treat the blood. For instance, the dialysate can be made concurrently or prior to dialysis, and stored within a dialysate storage vessel or the like.

Within the dialyzer, the dialysate and the blood typically are separated by a semipermeable membrane. Typically, the semipermeable membrane is formed from a polymer such as cellulose, polyarylethersulfone, polyamide, polyvinylpyrrolidone, polycarbonate, polyacrylonitrile, or the like, which allows the transport of ions or small molecules (e.g., urea, water, etc.), but does not allow bulk transport or convection during treatment of the blood. In some cases (such as high-flux dialyzer), even larger molecules, such as beta-2- microglobulin, may pass through the membrane. In some cases, for example, ions and molecules may pass through the dialyzer by convective flow if a hydrostatic pressure difference exists across the semi-permeable membrane.

It should be noted that, as used herein, "fluid" means anything having fluidic properties, including but not limited to, gases such as air, and liquids such as water, aqueous solution, blood, dialysate, etc.

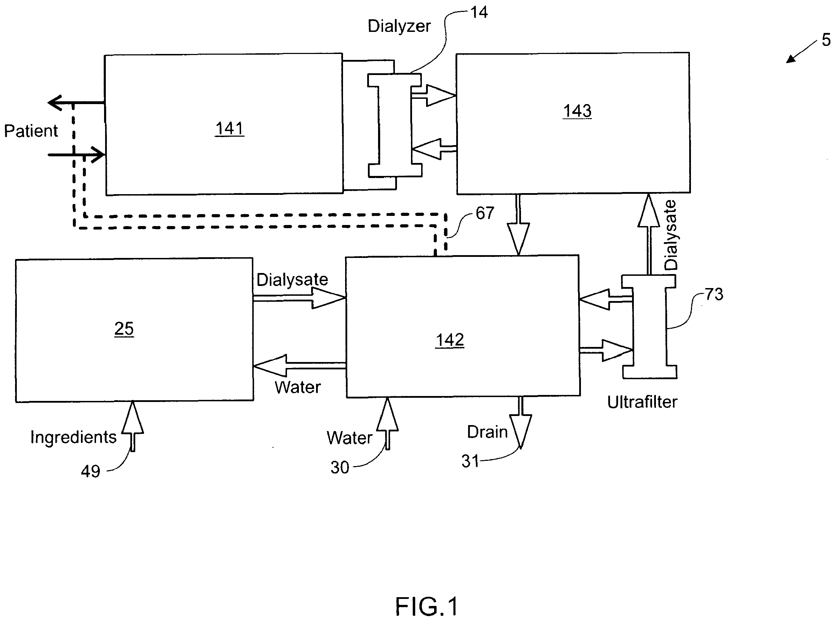

FIG. 1 shows a schematic block diagram of fluid circuitry for a hemodialysis system. In this illustrative system, the dialysis system 5 includes a blood flow circuit 141 that draws blood from a patient, passes the blood through a dialyzer 14, and returns the treated blood to the patient. A balancing circuit or an internal dialysate circuit 143 receives dialysate from an ultrafilter 73, passes the dialysate through the dialyzer 14, and receives used dialysate from the dialyzer 14. A directing circuit or an external dialysate circuit 142 provides fresh dialysate to the ultrafilter 73, and receives used dialysate from the internal dialysate circuit 143 (which may be directed to a drain 31). The directing circuit 142 can also receive water from a water supply 30 and pass it to a mixing circuit 25. The mixing circuit 25 forms dialysate using water from the directing circuit 142 and reagent ingredients 49, such as citric acid, salt and a bicarbonate, that may be received from a renewable source. The mixing circuit 25 may prepare dialysate, for example, on an as-needed basis, during and/or in advance of dialysis. New dialysate prepared by the mixing circuit 25 may be provided to the directing circuit 142, which may provide the dialysate to the ultrafilter 73, as described above. The directing circuit 142 may include a heater to heat the dialysate to a suitable temperature and/or to heat fluid in the system for disinfection. Conduits 67 (shown in dotted line) may be connected between the blood flow circuit 141 and the directing circuit 142, e.g., for disinfection of the hemodialysis system.

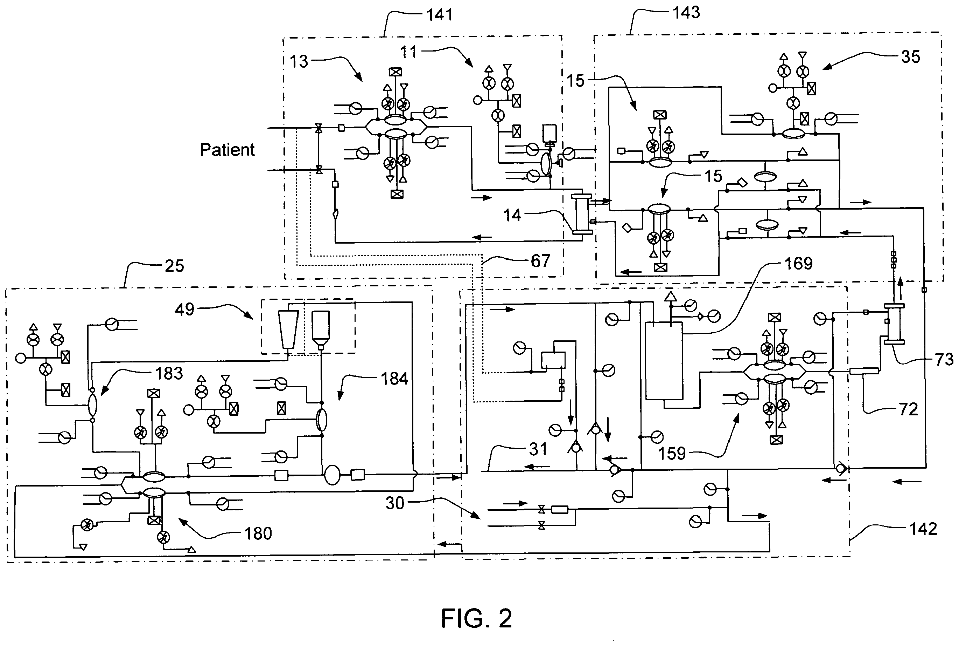

FIG. 2 is a schematic diagram showing a more detailed circuit arrangement for the dialysis system 5 shown in FIG. 1. It should be understood, of course, that FIG. 2 is only one possible example of the general hemodialysis system of FIG. 1, and in other embodiments, other fluid circuits, modules, flow paths, layouts, etc. are possible.

The blood flow circuit 141 includes an anticoagulant supply 11 and a blood flow pump 13 which pumps blood from a patient through a dialyzer 14 and returns the blood to the patient. The anticoagulant supply 11, although shown in the path of blood flowing towards the dialyzer, may be instead located in another suitable location, e.g., any location upstream or downstream from blood flow pump 13. The balancing circuit 143 includes two dialysate pumps 15, which pump dialysate into the dialyzer 14, and a bypass pump 35. The flow of blood through the blood flow circuit 141 in some cases, is synchronized with the flow of dialysate in the dialysate flow path. In one example, the flow of dialysate into and out of the dialyzer 14 and the balancing circuit 143 is balanced volumewise using balancing chambers in the balancing circuit 143. The directing circuit 142 includes a dialysate pump 159, which pumps dialysate from a dialysate tank 169 through a heater 72 and/or the ultrafilter 73 to the balancing circuit 143. The directing circuit 142 also receives waste fluid from balancing circuit 143 and directs it to a drain 31. In some cases, the blood flow circuit 141 can be connected via conduits 67 to the directing circuit 142, e,g., for disinfection, as discussed below. Dialysate in the dialysate tank 169 is provided by the mixing circuit 25, which produces the dialysate using water from a water supply 30 provided via the directing circuit 142 and dialysate ingredients 49 (e.g., bicarbonate and acid). A series of mixing pumps 180, 183, 184 are used to mix the various components and produce the dialysate.

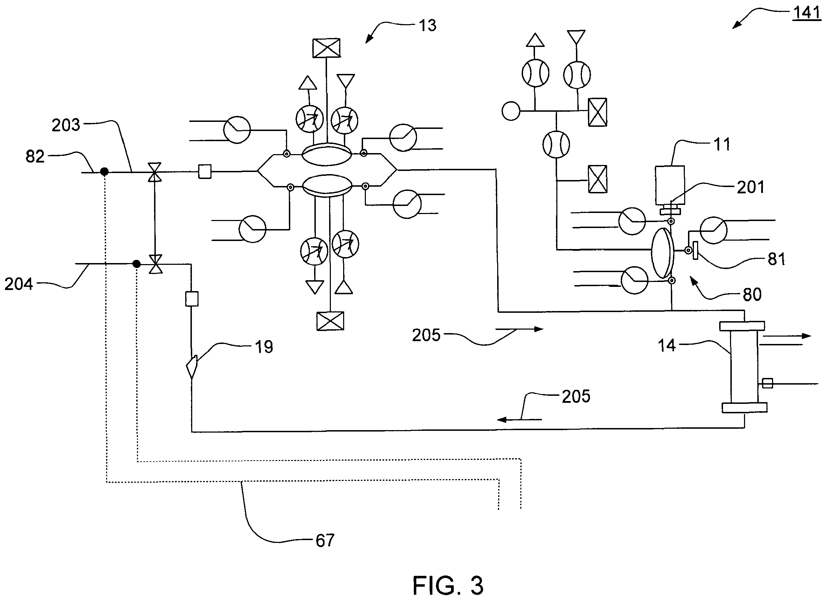

FIG. 3 shows a close-up view of the blood flow circuit 141 in this illustrative embodiment. Under normal operation, blood flows from a patient through arterial line 203 via blood flow pump 13 to the dialyzer 14 (the direction of flow during normal dialysis is indicated by arrows 205; in some modes of operation, however, the flow may be in different directions, as discussed below). Optionally, an anticoagulant may be introduced into the blood via anticoagulant pump 80 from an anticoagulant supply. After passing through dialyzer 14 and undergoing dialysis, the blood returns to the patient through venous line 204, optionally passing through an air trap and/or a blood sample port 19. The pump 13 may include, for instance, pumps 23 that are actuated by a control fluid.

For example, the blood flow pump 13 may comprise two (or more) pod pumps 23. Each pod pump, in this particular example, may include a rigid chamber with a flexible diaphragm or membrane dividing each chamber into a pumping compartment and control compartment. There may be four entry/exit valves for these compartments, two for the pumping compartment and two for the control compartment. The valves for the control compartment of the chambers may be two-way proportional valves, one connected to a first control fluid source (e.g., a high pressure air source), and the other connected to a second control fluid source (e.g., a low pressure air source) or a vacuum source. The fluid valves can be opened and closed to direct fluid flow when the pod pumps 23 are operating.

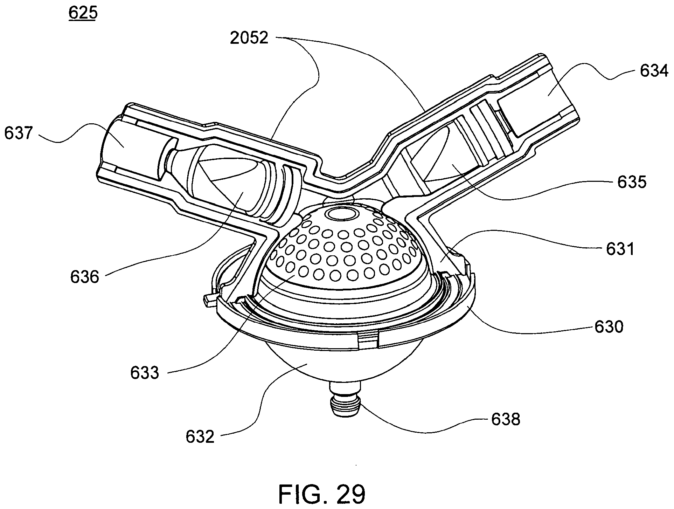

FIG. 29 shows one example of a reciprocating positive-displacement pump 625 that can be used in embodiments of the present disclosure. In this example, the reciprocating positive- displacement pump 625 is essentially a self-contained unit (which may be referred to hereinafter as a "pod") that may be used as a component of a larger pumping system. The reciprocating positive-displacement pump 625 includes a "top" portion (also referred to as the "pumping chamber wall") 631 and a "bottom" portion (also referred to as the "control chamber wall") 632 that are coupled together at pod wall 630, for example, by ultrasonic welding or other technique. It should be noted that the terms "top" and "bottom" are relative and are used here for convenience with reference to the orientation shown in FIG. 29. Each of the portions 631 and 632 has a rigid interior surface that is preferably (although not necessarily) hemispherical, such that the pod has an interior cavity that is preferably (although not necessarily) spherical.

In the example shown in FIG. 29, the control chamber wall 632 is a unitary structure while the pumping chamber wall 631 is formed from two halves that are coupled together along perimeter 2052, for example, by ultrasonic welding or other technique (which facilitates assembly of the integral valves, discussed below). Where the two portions of the pump chamber are molded, this design may allow for minimum flash or burrs, which is more likely to present a gentler pumping environment. This example may be advantageous for use with fluids vulnerable to shear forces, and where flash or burrs therefore should be avoided.

During typical fluid pumping operations, the application of negative pneumatic pressure to the pneumatic interface 638 tends to withdraw the membrane 633 toward the control chamber wall 632 so as to expand the pumping chamber and draw fluid into the pumping chamber through the inlet 634, while the application of positive pneumatic pressure tends to push the membrane 633 toward the pumping chamber wall 631 so as to collapse the pumping chamber and expel fluid in the pumping chamber through the outlet 637. During such pumping operations, the interior surfaces of the pumping chamber wall 631 and the control chamber wall 632 limit movement of the membrane 633 as it reciprocates back and forth. In the pump shown in FIG. 29, the interior surfaces of the pumping chamber wall 631 and the control chamber wall 632 are rigid, smooth, and approximately hemispherical. In lieu of a rigid control-chamber wall 632, an alternative rigid limit structure - for example, a portion of a bezel used for providing pneumatic pressure and/or a set of ribs - may be used to limit the movement of the membrane as the pumping chamber approaches maximum value. Thus, the rigid limit structure - such as the rigid control chamber wall 632, a bezel, or a set of ribs - defines the shape of the membrane 633 when the pumping chamber is at its maximum value. In a preferred example, the membrane 633 (when urged against the rigid limit structure) and the rigid interior surface of the pumping chamber wall 631 define a spheroid pumping-chamber volume when the pumping chamber volume is at a maximum.

Thus, in the pump shown in FIG. 29, movement of the membrane 633 is limited by the pumping-chamber wall 631 and the control-chamber wall 632. As long as the positive and negative pressurizations provided through the pneumatic port 638 are strong enough, the membrane 633 will move from a position limited by the control-chamber wall 632 to a position limited by the pumping-chamber wall 631. When the membrane is forced against the control-chamber wall 632, the membrane and the pumping-chamber wall 631 define the maximum volume of the pumping chamber. When the membrane is forced against the pumping-chamber wall 631, the pumping chamber is at its minimum volume.

In a preferred example, the pumping-chamber wall 631 and the control-chamber wall 632 both have a hemispheroid shape so that the pumping chamber will have a spheroid shape when it is at its maximum volume. More preferably, the pumping-chamber wall 631 and the control-chamber wall 632 both have a hemispherical shape so that the pumping chamber will have a spherical shape when it is at its maximum volume. By using a pumping chamber that attains a spheroid shape - and particularly a spherical shape - at maximum volume, circulating flow may be attained throughout the pumping chamber. Such shapes accordingly tend to avoid stagnant pockets of fluid in the pumping chamber. As discussed further below, the orientations of the inlet 634 and outlet 637 - with each being substantially tangential to the interior surface of the pumping chamber wall 631 - also tend to improve circulation of fluid through the pumping chamber and reduce the likelihood of stagnant pockets of fluid forming. Additionally, compared to other volumetric shapes, the spherical shape (and spheroid shapes in general) tends to create less shear and turbulence as the fluid circulates into, through, and out of the pumping chamber.

The pod pump components can be manufactured using any one of a number of methods of manufacturing, including but not limited to injection molding, compression molding, casting, thermoforming or machining. In some examples, where the pod pump chambers are machined, they can be fused together using mechanical fasteners or heat fused. In one embodiment of a disposable pump, the pump housing made from a thin film made of a material which includes, but is not limited to PETE, PETG, and PET. In these examples, the housing may be thermoformed, for example, vacuum or pressure formed, and the pump membrane is formed from a thin plastic film that can be heat sealed to the housing. In some examples, the pump housing is a multilayer film. These examples are conducive to bonding the pump housing to another component.

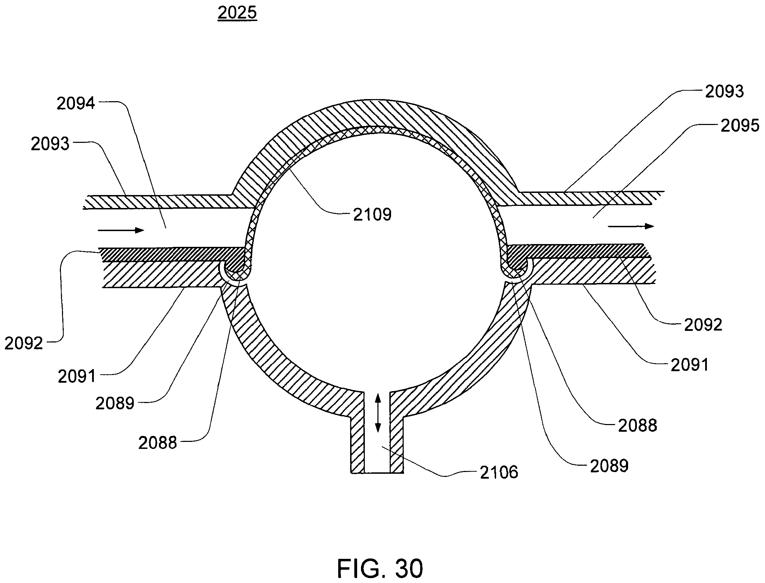

FIG. 30 is a sectional view of an alternative pod pump 2025 such as may be incorporated into a larger fluid-control cassette. In this example, the pod pump is formed from three rigid pieces, namely a "top" plate 2091, a middle plate 2092, and a "bottom" plate 2093 (it should be noted that the terms "top" and "bottom" are relative and are used here for convenience with reference to the orientation shown in FIG. 30). The top and bottom plates 2091 and 2093 may be flat on both sides, while the middle plate 2092 is provided with channels, indentations and holes to define the various fluid paths, chambers, and ports. To form the pod pump 2025, the top and bottom plates 2091 and 2093 may include generally hemispheroid portions that together define a hemispheroid chamber.

A membrane 2109 separates the central cavity of the pod pump into a chamber (the pumping chamber) that receives the fluid to be pumped and another chamber (the actuation chamber) for receiving the control gas that pneumatically actuates the pump. An inlet 2094 allows fluid to enter the pumping chamber, and an outlet 2095 allows fluid to exit the pumping chamber. The inlet 2094 and the outlet 2095 may be formed between middle plate 2092 and the bottom plate 2093. Pneumatic pressure is provided through a pneumatic port 2106 to either force, with positive gas pressure, the membrane 2109 against one wall of pod pump's cavity to minimize the pumping chamber's volume (as shown in FIG. 30), or to draw, with negative gas pressure, the membrane towards the other wall of the pod pump's cavity to maximize the pumping chamber's volume.

The membrane 2109 is provided with a thickened rim 2088, which is held tightly in a groove 2089 in the middle plate 2092. Thus, the membrane 2109 can be placed in and held by the groove 2089 before the top plate 2091 is ultrasonically welded to the middle plate 2092, so the membrane will not interfere with the ultrasonic welding of the two plates together, and so that securing the membrane does not depend on the two plates being ultrasonically welded together with exacting precision. Thus, it should be possible to manufacture this pod pump without requiring very tight tolerances during ultrasonic welding.

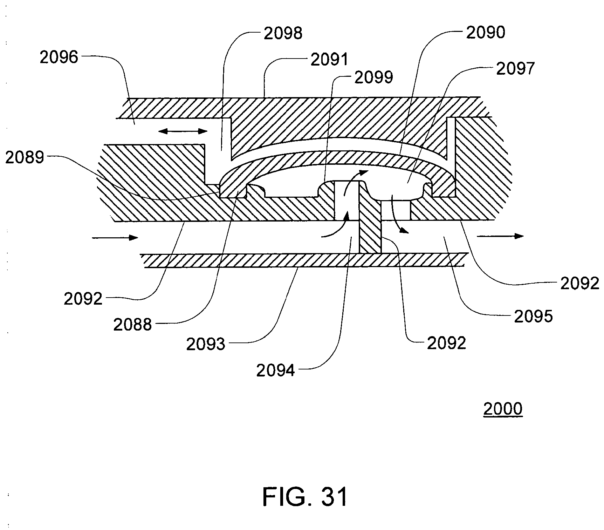

One or more pod pumps 2025 may be incorporated into a single cassette, which may also include one or more valves 2000. FIG. 31 is a sectional view of a pneumatically controlled valve 2000 that may be used in embodiments of the above-mentioned cassette. A membrane 2090, along with the middle plate 2092, defines a valving chamber 2097. Pneumatic pressure is provided through a pneumatic port 2096 to either force, with positive gas pressure, the membrane 2090 against a valve seat 2099 to close the valve, or to draw, with negative gas pressure, the membrane away from the valve seat to open the valve. A control gas chamber 2098 is defined by the membrane 2090, the top plate 2091, and the middle plate 2092. The middle plate 2092 has an indentation formed on it, into which the membrane 2090 is placed so as to form the control gas chamber 2098 on one side of the membrane and the valving chamber 2097 on the other side.

The pneumatic port 2096 is defined by a channel formed on the "top" surface of the middle plate 2092, along with the top plate 2091. By providing fluid communication between several valving chambers in a cassette, valves can be ganged together so that all the valves ganged together can be opened or closed at the same time by a single source of pneumatic pressure. Channels formed on the "bottom" surface of the middle plate 2092, along with the bottom plate, define the valve inlet 2094 and the valve outlet 2095. Holes formed through the middle plate 2092 provide communication between the inlet 2094 and the valving chamber 2097 (through the valve seat 2099) and between the valving chamber and the outlet 2095.

The membrane 2090 is provided with a thickened rim 2088, which fits tightly in a groove 2089 in the middle plate 2092. Thus, the membrane 2090 can be placed in and held by the groove 2088 before the top plate 2091 is ultrasonically welded to the middle plate 2092, so the membrane will not interfere with the ultrasonic welding of the two plates together, and so that securing the membrane does not depend on the two plates being ultrasonically welded together with exacting precision. Thus, it should be possible to manufacture this valve without requiring very tight tolerances during ultrasonic welding. As shown in FIG. 31, the top plate 2091 may include additional material extending into control gas chamber 2098 so as to prevent the membrane 2090 from being urged too much in a direction away from the groove 2089, so as to prevent the membrane's thickened rim 2088 from popping out of the groove 2089.

Although in this example, the pod pump is spheroid shaped, in still other examples, the pod pump can be any shape desired, such as an ovoid shape. Many of the embodiments of the pod pumps will include a pump chamber, an actuation chamber, a diaphragm (or movable member), at least one actuation port and at least one inlet/outlet port. In some examples, the pod pump includes an inlet and an outlet port. Various examples are described herein and features described with respect to one example should be understood to be available for any example, thus the example features can be mixed and matched, and any example can include one or more of the features described herein.

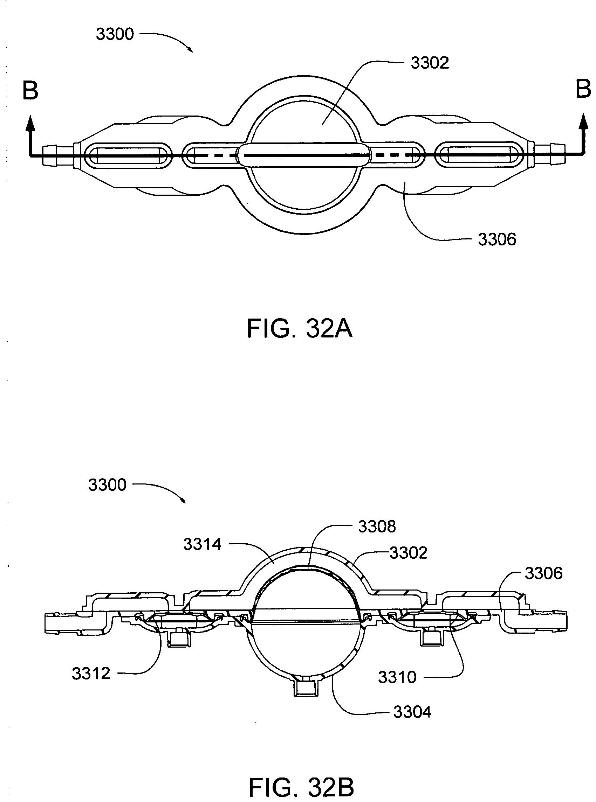

Referring to FIGS. 32A and 32B, an alternate example of a pod pump 3300 is shown with a pump chamber cover 3302, an actuation chamber cover 3304 and a mid plate portion 3306. In this example the mid plate 3306 and the actuation chamber cover 3304 retain the diaphragm 3308 and one or more secondary diaphragms 3310 or 3312. The secondary diaphragms may act passively or may be actuated by gas, liquid or mechanical forces to serve as active valves to control the flow of fluid through the pump chamber cover fluid path 3314. In this example of the pod pump 3300, a fluid path 3314 is formed in the pump chamber cover 3302 such that fluid may flow through the flow path 3314 regardless of the position of the diaphragm 3308. In this example as in other examples the pump chamber cover 3302, actuation chamber cover 3304 and mid plate 3306 are made of plastic, but in other embodiments may be made from other materials including but not limited to metal or glass. In one exemplary pump, covers 3302 and 3304, and mid plate 3306 are made from polysulfone. In another exemplary pump, they are made from medical grade polycarbonate. In this example the pump chamber cover 3302, actuation chamber cover 3304 and mid plate 3306 may be joined by laser welding or may be joined by various other methods as deemed appropriate for the chosen component materials and the desired pod pump use. Other joining possibilities include but are not limited to snap together tabs, press fit, snap fit, solvent bonding, heat welding, electromagnetic welding, resistance welding, RF welding, screws, bolts, ultrasonic welding, adhesive, clamping by components that neighbor the pump when in use or other joining methods commonly used in the art.

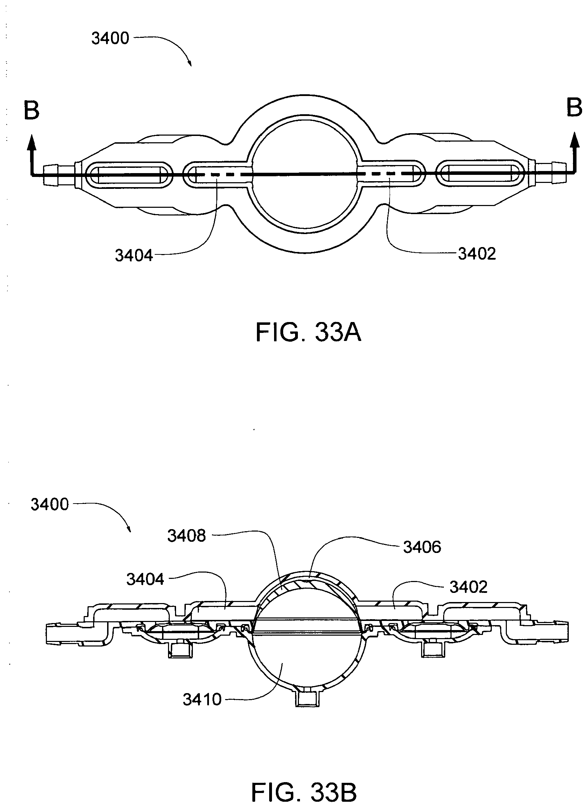

Referring now to FIGS. 33A and 33B one example of a pod pump 3400 is shown. In this pump, inlet and outlet ports are located at opposite ends of the pump chamber 3406 and are interchangeable depending on the configuration of the pump or its intended use. The diaphragm 3408 is shown nearly fully extended into the pump chamber 3406. In this example the inlet and outlet ports 3402 and 3404 may be partially or fully obscured by the diaphragm 3408 when fully actuated by fluid pressure in the actuation chamber 3410. Blocking of the inlet or outlet ports may serve to limit or switch the flow of subject fluid through the pump chamber 3406 as may be desired in certain applications. In this example the pumping side of the diaphragm 3408, i.e., the side of the diaphragm 3408 that contacts the subject fluid, is smooth, which may provide different flow characteristics with some subject fluids or provide different contact between the diaphragm 3408 and pump chamber 3406 when reduction of flow through the inlet or outlet ports 3402 and 3404 is desired when the diaphragm is fully extended into the pump chamber 3406.

In some embodiments, the diaphragm has a variable cross-sectional thickness, as shown in FIG. 33B. Thinner, thicker or variable thickness diaphragms may be used to accommodate the strength, flexural and other properties of the chosen diaphragm materials. Thinner, thicker or variable diaphragm wall thickness may also be used to manage the diaphragm thereby encouraging it to flex more easily in some areas than in other areas, thereby aiding in the management of pumping action and flow of subject fluid in the pump chamber 3406. In this pump the diaphragm 3408 is shown having its thickest cross-sectional area closest to its center. However in other pumps having a diaphragm 3408 with a varying cross-sectional thickness, the thickest and thinnest areas may be in any location on the diaphragm 3408. Thus, for example, the thinner cross-section may be located near the center and the thicker cross-sections located closer to the perimeter of the diaphragm 3408. Still other configurations are possible.

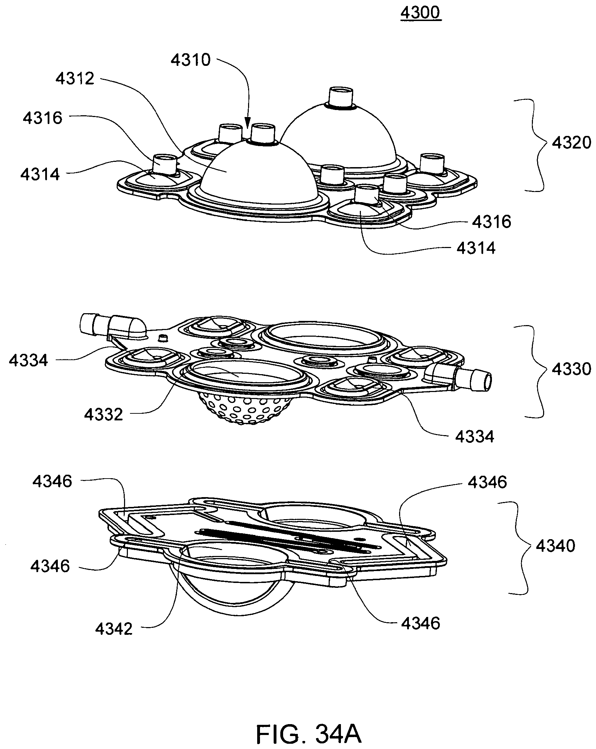

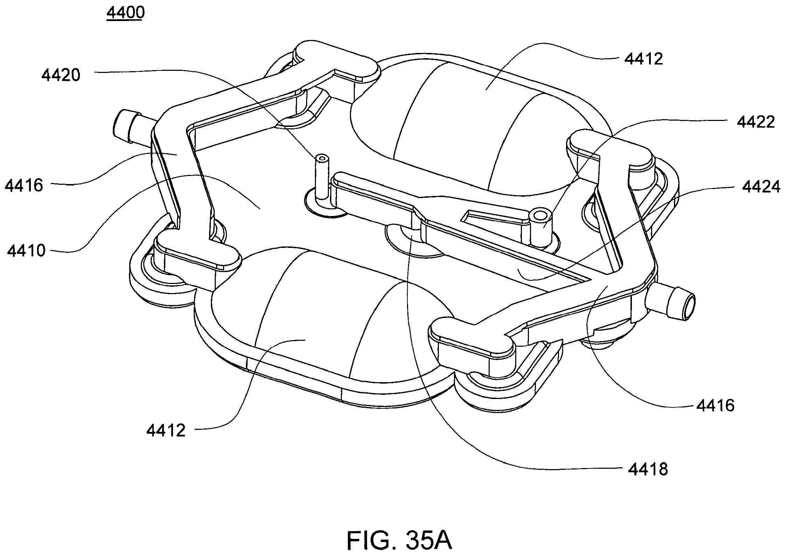

In some examples, the pod pump is incorporated into a device which is then integrated or attached to a machine, device, or container. One example of this is a cassette having integrated pod pumps, fluid paths, fluid ports, actuation ports and actuation fluid paths. Two examples of a cassette are described with respect to FIGS. 34A-34C and 35A-35B. It is understood that these are merely exemplary cassettes, and that many additional embodiments having different numbers and arrangements of pumps, valves and flow paths can be constructed using similar principles.

Referring now to FIGS. 34A-34C, a pod pump cassette 4300 is shown. Referring now to FIG. 34A, this pod pump cassette includes two pod pumps 4310. The pod pumps 4310 can be any type of pod pump, but in this exemplary cassette, the pod pumps 4310 are similar to the pod pump shown in FIGS. 32 and 33. The cassette 4300 includes three plates, an actuation plate 4320, a mid plate 4330 and a pump chamber plate 4340.

The actuation plate 4320 includes, for each pod pump 4310, a pod pump actuation chamber housing 4312 portion and two valves actuation housing 4314 portions. The valve actuation housing 4314 includes a valve actuation port 4316. In addition to pod pumps, the cassette 4300, in some examples, may contain additional ports and/or containers to or from which various fluids can be pumped.

The mid plate 4330 includes, for each pod pump, a pump diaphragm 4332 and two valve diaphragms 4334. In the embodiment shown, the valves are volcano or active valves actuated by a diaphragm 4334 which is actuated by a fluid, which in this cassette is pneumatic air. Also shown on this cassette 4300 are additional diaphragms in the mid plate 4330.

Referring now to the pump plate 4340, each pod pump 4310 includes a pump chamber housing 4342 which includes an integral fluid path 4344. The pump chamber housing 4342 is in fluid connection with an exterior fluid path 4346.

In this example, the three plates 4320, 4330, 4340 are laser welded together. However, in other examples, various modes of attachment, some of which are described above, may be used.

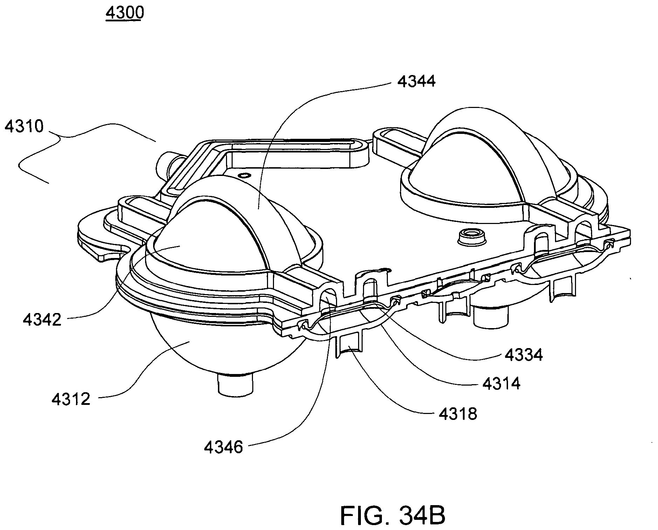

Referring now to FIG. 34B, a cross sectional view of the cassette 4300 is shown. The volcano valves are shown including the valve diaphragms 4334, the valves actuation housing 4314 portions and the exterior fluid line 4346. The valves are actuated by pneumatic air through actuation ports 4318.

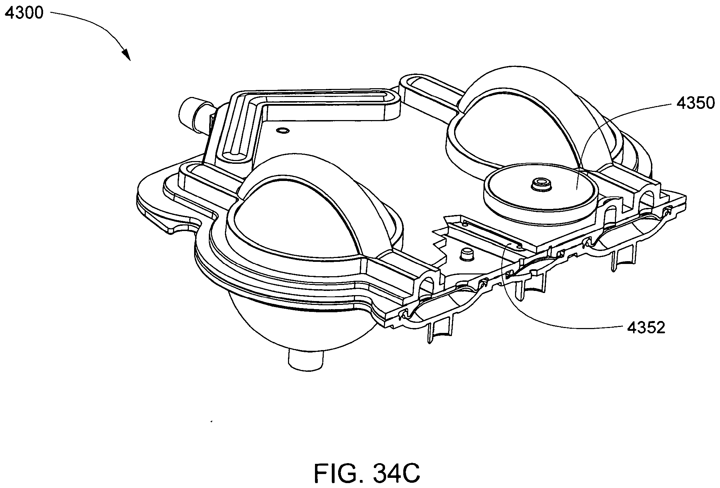

Referring now to FIG. 34C, in some examples, an air filter 4350 and an additional fluid line 4352 may be included in the cassette.

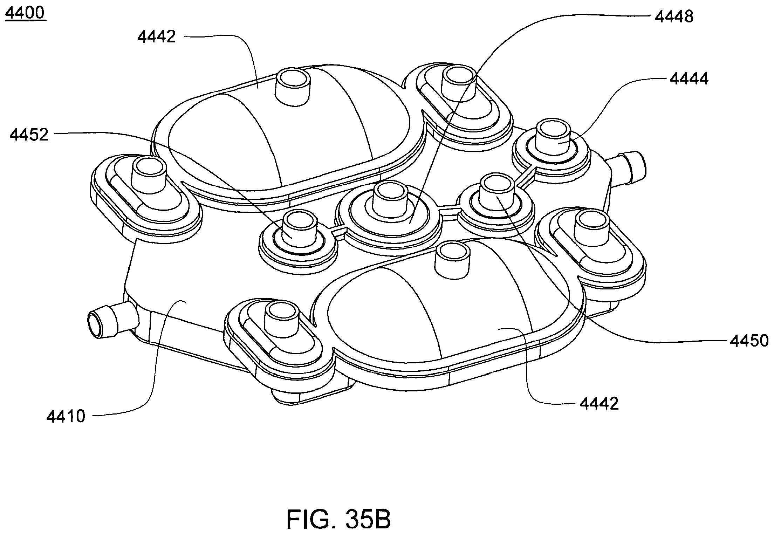

An alternate example of the cassette is shown in FIGS. 35A and 35B. Referring now to FIG. 35A, the cassette 4400 includes greater than three portions. The portions include a mid plate 4410 with multiple covers 4412-4416 laser welded onto the mid plate. These multiple covers 4412-4416 are used rather than the pump plate shown in FIG. 34A as 4340. Referring now to FIG. 35B, the mid plate 4410 again is shown. However, in this cassette, multiple covers 4442-4444 are used rather than a single actuation plate as shown in FIG. 34A as 4320. FIGS. 35A-35C show one example; in other examples, the number of multiple covers may very.

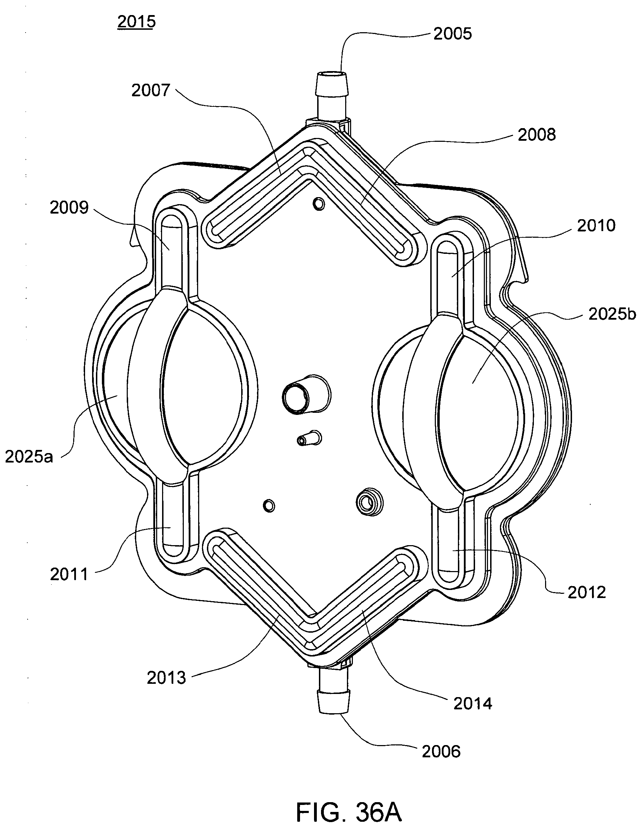

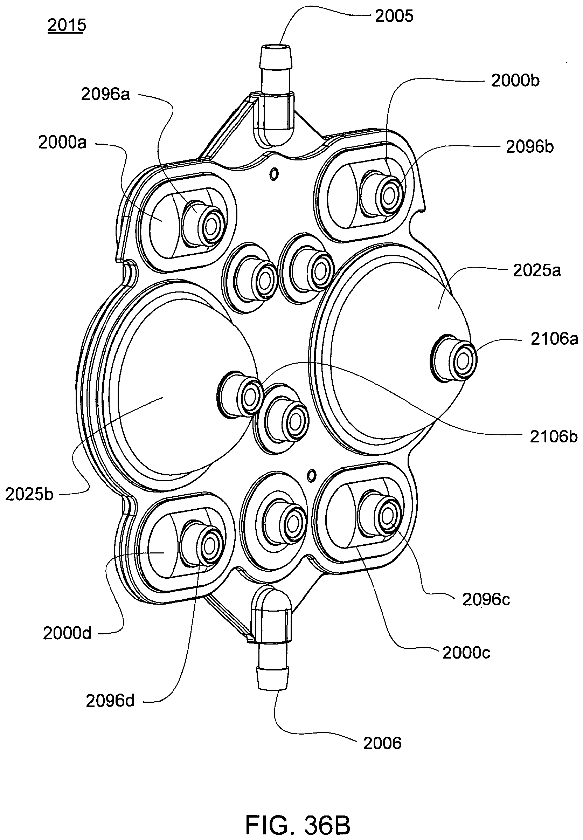

As shown in FIGS. 36A and 36B, two pod pumps 2025a and 2025b of the type shown in FIG. 34A-34C and a number of valves 2000a - 200Od of the type shown in FIG. 31 are incorporated in a pump cassette 2015 along with various fluid paths and other components. The pump cassette 2015 includes a common inlet 2005 in fluid communication with pod pump 2025a via fluid paths 2007 and 2009 and with pod pump 2025b. via fluid paths 2008 and 2010. The pump cassette 2015 also includes a common outlet 2006 in fluid communication with pod pump 2025a via fluid paths 2011 and 2013 and with pod pump 2025b via fluid paths 2012 and 2014. Thus, pod pumps 2025a and 2025b draw fluid from the common inlet 2005 and pump fluid to the common outlet 2006. That being said, valve 2000a is used to control fluid flow at the intersection of fluid paths 2008 and 2010 (i.e., at the inlet to pod pump 2025b); valve 2000b is used to control fluid flow at the intersection of fluid paths 2007 and 2009 (i.e., at the inlet to pod pump 2025a); valve 2000c is used to control fluid flow at the intersection of fluid paths 2011 and 2013 (i.e., at the outlet of pod pump 2025a); and valve 200Od is used to control fluid flow at the intersection of fluid paths 2012 and 2014 (i.e., at the outlet of pod pump 2025b). Each of the pod pumps 2025a and 2025b has its own pneumatic interface 2106a and 2106b, respectively. Also, each of the valves 2000a-2000d has its own pneumatic interface 2096a-2096d, respectively. Thus, each of pod pumps and each of the valves can be independently controlled by a base station.

If more than one pod pump is present, the pod pumps may be operated in any suitable fashion, e.g., synchronously, asynchronously, in-phase, out-of-phase, etc. For instance, in some examples, the two-pump pumps can be cycled out of phase to affect the pumping cycle, e.g., one pump chamber fills while the second pump chamber empties. A phase relationship anywhere between 0° (the pod pumps fill and empty in unison) and 180° (one pod pump fills as the other empties) can be selected in order to impart any desired pumping cycle. A phase relationship of 180° may yield continuous flow into and out of the set of pod pumps. This is useful, for instance, when continuous flow is desired, e.g., for use with dual needle or dual lumen catheter flow. Setting a phase relationship of 0°, however, may be useful in some cases for single needle/single lumen flow or in other cases. In a 0° relationship, the pod pumps will first fill from the needle, then deliver blood through the blood flow path and back to the patient using the same needle. In addition, running at phases between 0° and 180° can be used in some cases, to achieve a push/pull relationship (hemodiafiltration or continuous back flush) across the dialyzer.

An anticoagulant (e.g., heparin, or any other suitable anticoagulant) may be contained within a vial 11 (or other anticoagulant supply, such as a tube or a bag), and blood flow circuit 141 may include a spike 201 (which, in one embodiment, is a needle) that can pierce the seal of the vial. The spike 201 may be formed from plastic, stainless steel, or another suitable material, and may be a sterilizable material in some cases, e.g., the material may be able to withstand sufficiently high temperatures and/or radiation so as to sterilize the material.

An anticoagulant pump 80, which can act as a metering chamber in some cases, can be used to control the flow of anticoagulant into the blood circuit. The anticoagulant pump 80 may be a pod pump or a membrane-based metering pump, and/or may be actuated by a control fluid, such as air. For example, the anticoagulant pump 80 may include a rigid chamber with a flexible diaphragm dividing the chamber into a pumping compartment and a control compartment. One valve for the control compartment of the chamber may be connected to a first control fluid source (e.g., a high pressure air source), and the other valve connected to a second control fluid source (e.g., a low pressure air source) or a vacuum source. Valves for the pumping compartment of the chamber can be opened and closed in coordination with the control compartment, thus controlling the flow of anticoagulant into the blood. In one set of examples, air provided through a filter 81 may also be introduced into the blood flow path by the anticoagulant pump 80, e.g., to provide air into the vial 11 after or before anticoagulant is withdrawn from the vial.

Fluid Management System ("FMS") measurements may be used to measure the volume of fluid pumped through a pump chamber during a stroke of the membrane, or to detect air in the pumping chamber. In one illustrative example, the volume of liquid delivered by an anticoagulant pump, a dialysate pump, or other membrane-based fluid pump is determined using an FMS algorithm in which changes in chamber pressure are used to calculate a volume measurement at the end of a fill stroke and at the end of a delivery stroke. The difference between the computed volumes at the end of fill and delivery strokes may be used to determine the actual stroke volume. This actual stroke volume can be compared to an expected stroke volume for the particular sized chamber. If the actual and expected volumes are significantly different, the stroke has not properly completed and an error message can be generated.

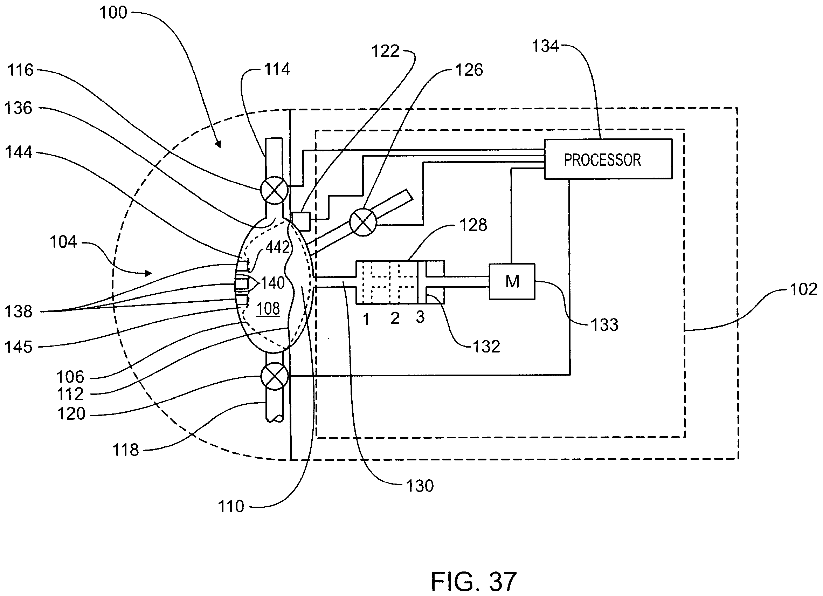

One pumping system that utilizes a measurement gas to actuate a pump chamber to pump a liquid and to detect the presence of a gas in the pumping chamber is shown schematically in FIG. 37. Pumping system 100 includes a fluid supply system 102 containing a fixed quantity of a measurement gas and a mechanism for changing the volume of the measurement gas within the system. Pumping system 100 also includes a pump 104 comprising a substantially rigid container 106 that includes a pump chamber 108 and a control chamber 110 disposed therein. Pump chamber 108 and control chamber 110 are fluidically isolated (i.e., not able to be placed in fluid communication) from each other by a flexible membrane 112, disposed between the two chambers, such that pump chamber 108 is coupled to control chamber 110 and in operative association therewith. Such a membrane may (as just one example) be constructed of medical grade polyvinyl chloride.

"Substantially rigid" as used herein refers to a material, or a component constructed therefrom, that does not flex or move substantially under the application of forces applied by the pumping system. A "control chamber" as used herein refers to a chamber of a pumping system that is coupled to, or contains, a volumetric chamber, for example a pump chamber, for the purpose of exerting a force on the volumetric chamber and, in preferred embodiments, for determining a measured parameter related to the volume of the volumetric container. The term "coupled to" as used in this context with respect to chambers or other components of the pumping system, refers to the chambers or components being attached to, or interconnected with, another component of the pumping system, such that the other component is able to exert a force on an external surface of the chamber or component to which it is coupled.

Liquid to be pumped by pump system 100 enters pump chamber 108 via inlet line 114 including an inlet valve 116. Liquid can be pumped from pump chamber 108 to a desired downstream destination through outlet line 118 including an outlet valve 120 therein. Control chamber 110 includes a pressure measuring component 122 for determining the pressure of the measurement gas within the control chamber. A "pressure measuring component" as used herein refers to a device that is able to convert a fluid pressure into a measurable signal or parameter. Pressure measuring components that may be useful in this example include but are not limited to: transducers; pressure gauges; manometers; piezoresistive elements; and others as apparent to those of ordinary skill in the art.

Control chamber 110 of pumping system 100 may also include a vent line 124 including a vent valve 126 therein. Control chamber 110 is connected in fluid communication with a variable volume cylinder 128 via a measurement gas inlet line 130. Variable volume cylinder 128 which includes a piston 132 therein which is moved and actuated by motor 133 for compressing, or expanding the volume of the measurement gas contained within the system.

Pumping system 100 also preferably contains a processor 134 which is in electrical communication with the various valves, pressure transducers, motors, etc. of the system and is preferably configured to control such components according to a desired operating sequence or protocol. Reference to a processor being "configured" to perform certain tasks herein refers to such processor containing appropriate circuitry, programming, computer memory, electrical connections, and the like to perform a specified task. The processor may be implemented as a standard microprocessor with appropriate software, custom designed hardware, or any combination thereof. As discussed in more detail below, processor 134, in addition to including control circuitry for operating various components of the system, also preferably includes a comparer that is configured to determine a measured parameter related to the volume of pump chamber 108 and to detect the presence of any gas contained within pump chamber 108 during operation of pump 104. A "comparer" as used herein refers to a processor (e.g., with appropriate programming) or circuit or component thereof that is able to compare the values of two or more measured parameters or other parameters derived therefrom.

In cases where allowing gas to migrate through the system is problematic, pump chamber 108 is oriented in an essentially vertical configuration during operation such that inlet line 114 is disposed above outlet line 118. The above-described orientation is advantageous for preventing any gas which may be present in pump chamber 108 during operation from being pumped from the pump chamber to a downstream destination through outlet line 118. Instead, any gas contained within pump chamber 108 will tend to rise towards the top of the pump chamber, for example the region adjacent to inlet port 136, and will be detected by the system, as described in more detail below, before being pumped from the pump chamber.

In some examples, pump chamber 108 includes the novel inclusion of a plurality of spacers 138 included therein. The spacers 138 function to prevent flexible membrane 112 from contacting an inner surface 140 of the pump chamber when the liquid contained within pump chamber 108 is being pumped through outlet line 118. During the pump stroke, the maximum displacement of flexible membrane 112 which is permitted by spacers 138 is shown in FIG. 37 by dashed line 442. It can be seen that even with flexible membrane 112 at its maximum displacement into pump chamber 108, as defined by dashed line 442, spacers 138 create a dead space 144 to contain any gas which may be present in pump chamber 108, thus inhibiting the gas from being pumped through the pump chamber. Spacers 138, in combination with the vertical orientation of pump chamber 108, also serve to assist any gas present in pump chamber 108 to rise to the top of the pump chamber so that it may more easily be purged from the pump chamber, as described in more detail below.

Pump chamber 108 of pumping system 100 is essentially defined by a substantially rigid wall 145 (e.g., made of a rigid plastic such as a polyacrylate) having a flexible membrane 112 disposed over the wall, thus forming a volumetric chamber.

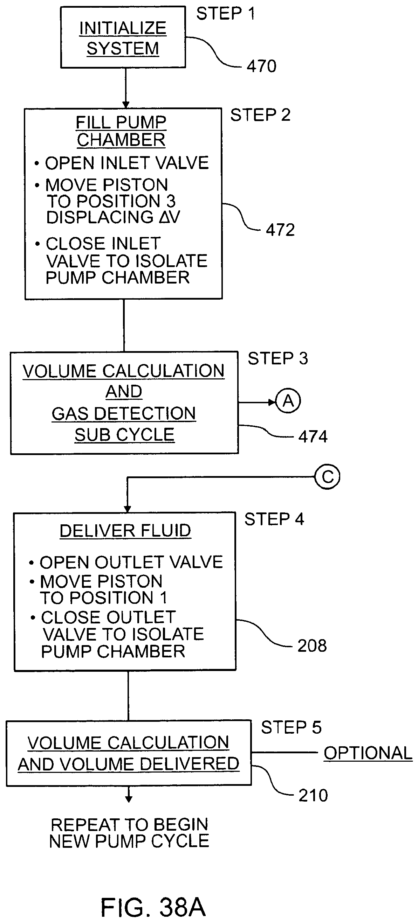

One method for operating the pumping system 100 shown in FIG. 37 for pumping a liquid with pump chamber 108, and for detecting the presence of a gas in pump chamber 108, is shown in detail in the flow charts of FIGS. 38A-38C. Referring to FIG. 38A, an exemplary pump cycle utilizing pumping system 100 will be described. The pump cycle illustrated utilizes changes in displacement of the piston to change the pressure of a measurement fluid within the system in order to apply selected forces to membrane 112 for pumping and air detection. The illustrated method also utilizes an equation of state (e.g. the ideal gas law) in determining pump chamber volumes from measured or known values of pressure and volume.

For examples employing a protocol for detecting air/gas where pump and/or control chamber volumes are determined, at least in part, from measured pressures by utilizing an equation of state describing the pressure-volume behavior of a measurement gas, the pump chamber preferably includes a movable surface which comprises an elastic membrane. The restoring force of the elastic membrane, when stretched or displaced from a relaxed equilibrium condition, enables the pressure on each side of the membrane (i.e. in the pump chamber and control chamber) to be different, where the degree of difference in the pressures, and the resistance to further displacement/stretching (stress/elastic energy stored in the membrane), is a function of the degree of stretch or displacement from the relaxed equilibrium condition of the membrane. In such examples, it is also preferred that the measurement gas pressures applied to the elastic membrane during the determination of pump/control chamber volumes at the first and second conditions of applied force for detecting air/gas in the pump chamber discussed above, tend to stretch the elastic membrane (if air/gas is present in the pump chamber), from its equilibrium configuration before the pressure is applied, by a different extent for each condition, so that the stress in the membrane and its resistance to further displacement in response to a given level of applied pressure will be different for the first and second condition (or in other words, the force/displacement response of the elastic membrane for the first and second conditions will be asymmetrical). In such examples, the difference in the pressure in the control chamber versus the pressure in the pump chamber, at an equilibrium condition, will be different for the first condition of applied pressure versus the second condition of applied pressure. In such examples, without being tied to any particular physical mechanism, it is believed that the different level of stress and strain of the elastic membrane during measurements of pump/control volume determined at the first and second conditions above create, at least in part, deviations in the pressure-volume behavior of the measurement gas from that predicted for each condition by the equation of state, which deviations can create and/or enhance a difference in the volume of the pump/control chamber determined for each condition by using the equation of state.

One way to achieve or enhance such asymmetry in the response of the elastic membrane to the applied measurement gas pressures utilized during volume determinations for gas detection is to perform the volume determination steps when the pump chamber flexible elastic membrane has already been stretched, from the configuration it has at a relaxed equilibrium condition, with essentially equal fluid pressures on each side of the membrane, before the application of pressurized measurement gas to the membrane for the purpose of volume measurements. This can be accomplished, for example, by performing the volume determinations related to air/gas detection after filling the pump chamber with sufficient liquid so that the elastic membrane is at least somewhat stretched, and preferably substantially stretched, by displacement of the membrane in the direction of the control chamber, and by using a positive measurement gas pressure during volume measurement at the first condition and a negative measurement gas pressure during volume measurement at the second condition (or vice- versa. In alternative embodiments the desired asymmetry in the response of the elastic membrane during volume determinations involved in air/gas detection could also be achieved by utilizing levels of measurements gas pressures applied to the elastic membrane for volumetric determinations performed at the first and second conditions of measurement that are selected to impart a different, and preferably substantially different degree of elastic stretch to the membrane. While preferred examples of pump chambers for use when utilizing an equation of state based procedure for calculating pump/control chamber volumes include a moveable surface at least partially comprised of an elastic membrane, in alternative embodiments, non-elastic movable surfaces could potentially be used, as long as the measurement fluid pressures applied to the surface during volume measurement at the first condition and second condition create different levels of stress in the surface and differences in the equilibrium pressures within the control and pump chamber. Such examples could, for example, utilize a non-elastic movable surface or flaccid membrane, where measurement fluid pressures applied during the first condition of volume determinations tend to move the surface/membrane (if a gas is present in the pump chamber) to its maximum allowed displacement so that the surface is no longer free to move in response to the applied force, a stress is created in the surface/membrane, and a pressure difference exists between the pump and control chambers. Measurement of volume at a second condition for such examples could apply a different measurement fluid pressure to the surface, the pressure tending to move the surface/membrane (if a gas is present in the pump chamber) to reduce or substantially eliminate the stress within the surface/membrane so that at equilibrium, the difference in pressure in the pump and control chambers is reduced or essentially eliminated.

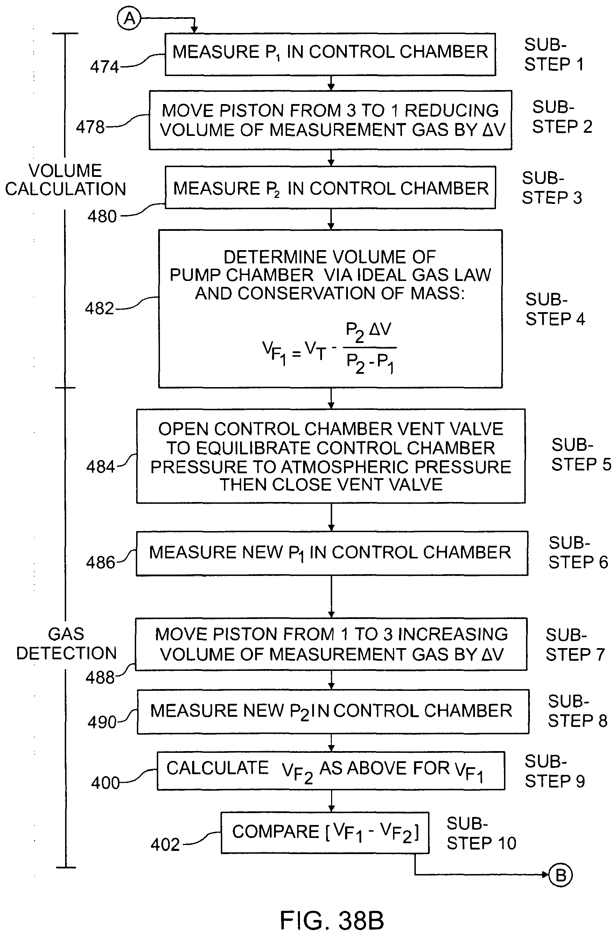

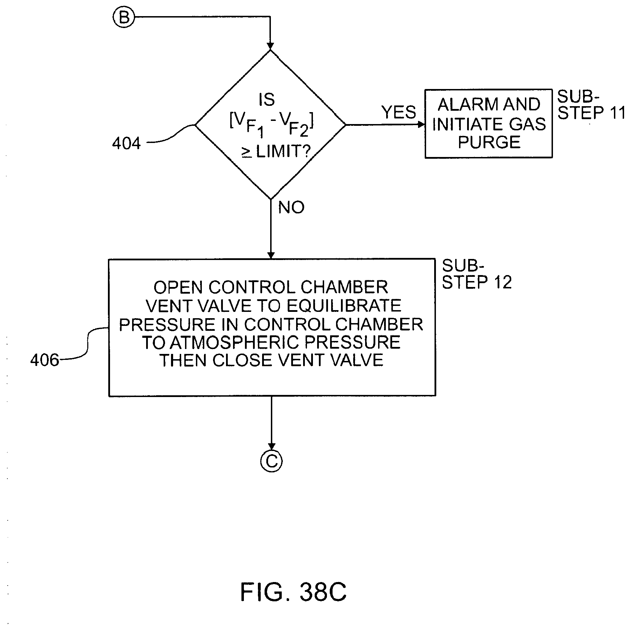

Referring again to the protocol of FIG. 38, it will be assumed initially that pump chamber 108 has been emptied, and that elastic membrane 112 is extending into pump chamber 108 at its maximum allowable displacement defined by line 442. Piston 132 is assumed to be at its far left position of travel (shown as position 1 in FIG. 37). Referring to FIG. 38A, step 1 (470) involves initializing the system so that all valves are closed and piston 132 and flexible membrane 112 are in the positions described above. Step 2 (472) involves filling the pump chamber 108 with a liquid to be pumped. The step involves first opening inlet valve 116, then actuating motor 133 so as to move piston 132 to position 3 Shown in FIG. 37, thereby increasing the volume of pump chamber 108 by an amount defined as .DELTA.V. Then, inlet valve 116 is closed in order to isolate pump chamber 108. Step 3 (474) of the exemplary pumping cycle involves a series of sub, and for detecting the presence of any gas contained within pump chamber 108. Step 3 (474) is described in greater detail in FIG. 38B. Referring again to FIG. 38A, step 4 (208) of the pumping cycle involves delivering the liquid contained in pump chamber 108. First, outlet valve 120 is opened. Motor 134 is then actuated to move piston 132 from position 3 to position 1, thereby delivering a volume of fluid .DELTA.V. Outlet valve 120 is then closed in order to isolate pump chamber 108. In some embodiments in which the accuracy of determining the volume delivered by pump chamber 108 is critical, the volume of pump chamber 108 after step 4 (208) may be determined, for example, by repeating substeps 1-4 (476, 478, 480, 482) of the volume calculation and air detection subcycle of FIG. 38B. In that case, the volume delivered for the above described pump stroke can be determined by taking a difference in the volume of pump chamber 108 determined in step 3 (474) and in step 5 (210). Finally, if multiple pump strokes are desired, the entire pump cycle of FIG. 38 A may be repeated.

FIGS. 38B-38C show one example of a volume calculation and gas detection method shown at step 3 (474) of FIG. 38A. Substep 1 (476) of subcycle 474 involves measuring the pressure P.sub.1 of the measurement gas in control chamber 110 with pressure transducer 122 and recording or storing the pressure with processor 134. In substep 2 (478) piston 132 is moved from position 3 to position 1 thereby reducing the volume of the measurement gas contained within the system by .DELTA.V. In substep 3 (480) the pressure of the measurement gas in control chamber 110 is measured again and recorded as P.sub.2. It will be appreciated that P.sub.2 will be greater than P.sub.1 due to the compression of measurement gas within the system. The volume of fluid contained in pump chamber 108 is then determined in substeps 4 (482), with the pump chamber at this first conditions, using an appropriate equation of state for the measurement fluid being utilized. In the case of a measurement gas, such as air, for systems utilizing pumping pressures which are relatively low (typical pumping pressures utilized by such pumping systems range from abut -14 psig to about 15 psig) the ideal gas law can be employed. Recognizing that no measurement gas was added to or removed from the system, and utilizing the ideal gas law combined with conservation of mass, the volume of fluid contained in pump chamber 108 is determined by Equation 1 (see FIG. 38B, Substep 4):

$$V_{F1} = V_T - (P^2 \Delta V)/(P_2 - P_1)$$

Equation 1 assumes that any temperatures changes or differences caused by changing the volume of measurement gas are minimal and that the system is essentially isothermal. It will be appreciated that for systems where temperature changes may be significant, the temperature dependence of the measurement fluid, as defined by the equation of state being used, may be incorporated into the volume calculation of substeps 4 (482) in a straightforward fashion, as apparent to those of ordinary skill in the art. V.sub.F in equation 1 refers to the internal volume of pump chamber 108 and V.sub.T refers to the known total volume of the system including pump chamber 108, control chamber 110, and the volumes contained within measurement fluid inlet line 130 and cylinder 128.

The remaining substeps of the volume calculation subcycle 474 involve re-determining the volume of the pump chamber 108 at a different condition and comparing the volumes determined at the first and second conditions. In substep 5 (484) of FIG. 38B, control chamber vent valve 126 is opened, to equilibrate the pressures in control chamber 110 with the surrounding atmosphere. Vent valve 126 is then closed. A new pressure P-sub-1 is measured with transducer 122 in control chamber 110 in substep 6 (486). In substep 7 (488) piston 132 is moved from position 1 to position 3 thereby increasing the volume of measurement gas within the system by .DELTA.V. In substep 8 (490) the new pressure P.sub.2 in control chamber 110, which will be below atmospheric pressure, is measured and recorded. In substep 9 (400) the volume of pump chamber 108 V.sub.F is calculated as described above in substep 4 (482). Substep 10 (402) involves determining the difference between V.sub.F determined in substep 4 (482) and V.sub.F determined in substep 9 (400) and taking an absolute value of the difference. In substep 11 (404), shown in FIG. 38C, the above difference is compared to a predetermined limit that is proportional to a maximum allowable quantity of air or other gas which can be present in pump chamber 108 during operation. The predetermined limit is typically determined empirically, and chosen such that air volume exceeding dead space 144 volume will also exceed the predetermined limit. If the difference exceeds the predetermined limit the processor 134 will create an alarm condition and initiate an air purge.

If the difference in measured volumes is less than the allowable limit (404), the system will proceed to pump the liquid contained in pump chamber 108. In substep 12 (406) the system opens control chamber vent valve 126 in order to equilibrate the pressure in control chamber 110 and the surrounding atmosphere, and then closes vent valve 126. Pumping system 100 is now in condition to deliver the liquid contained in pump chamber 108.

As described above, the measured volumes at the two different conditions can be compared to detect the presence of gas in the pump chamber. If the presence of a gas is detected in the pump chamber and is of sufficient quantity to cause the system to set off an alarm, as described above in substep 11 (404) FIG. 38C, instead of proceeding to deliver the fluid to a desired downstream destination as described above, the pumping system 100 will instead initiate an air purge. During the air purge, instead of outlet valve 120 being opened while fluid is being pumped from pump chamber 108, inlet valve 116 is opened, and the fluid, including any gas in the pump chamber, is pumped from the pump chamber through inlet line 114 to a safe purge destination.

The blood flow circuit 141 may also include an air trap 19 to remove air bubbles that may be present within the blood flow path. In some cases, the air trap 19 is able to separate any air that may be present from the blood due to gravity, and /or may include a port for sampling blood.

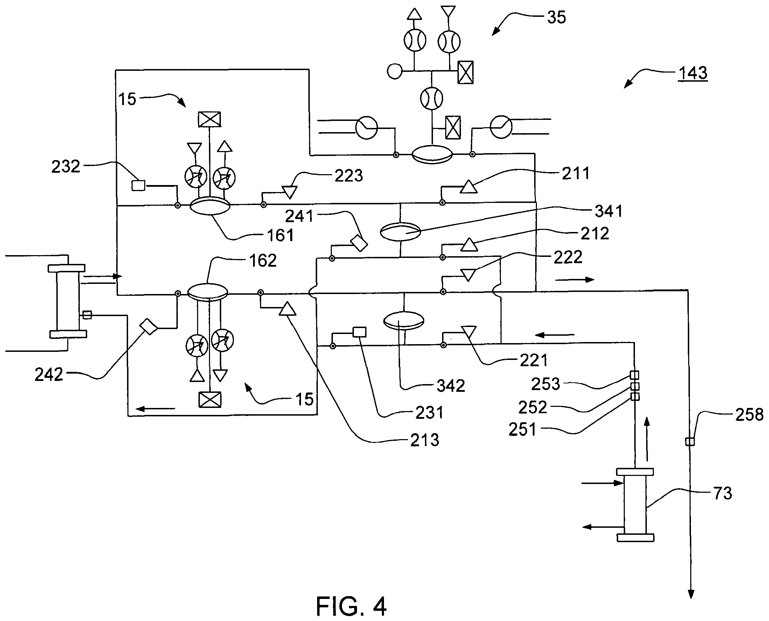

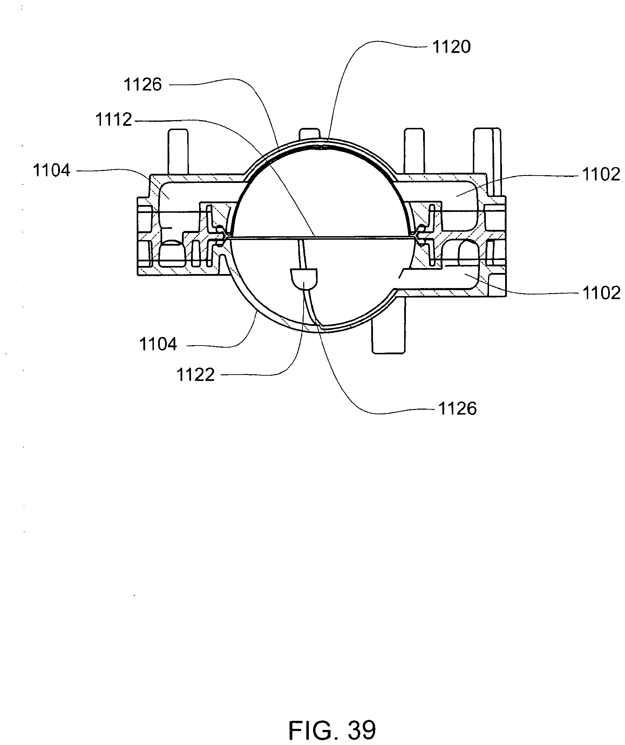

FIG. 4 shows a close-up view of the balancing circuit 143 in the FIG. 2 dialysis system. In the balancing circuit 143, dialysate flows from the optional ultrafilter 73 into a dialysate pump 15. In this example, the dialysate pump 15 includes two pod pumps 161, 162, two balancing chambers 341, 342, and a pump 35 for bypassing the balancing chambers 341, 342. The balancing chambers 341, 342 may be constructed such that they are formed from at rigid chamber with a flexible diaphragm dividing the chamber into two separate fluid compartment, so that entry of fluid into one compartment can be used to force fluid out of the other compartment and vice versa. An exemplary balancing pod is shown in FIG. 39. The balancing pod is constructed similarly to the pod pumps described above. However, a balancing pod includes two fluid balancing chambers, rather than an actuation chamber and a pumping chamber, and does not include an actuation port. Additionally, each balancing chamber includes an inlet 1102 and an outlet 1104. In the exemplary balancing pod, a groove 1126 is included on each of the balancing chamber walls 1120, 1122. The groove 1126 is described in further detail below. The membrane 1112 provides a seal between the two chambers. The balancing chambers work to balance the flow of fluid into and out of the chambers such that both chambers maintain an equal volume rate flow. Although the inlets 1102 and outlets 1104 for each chamber are shown to be on the same side, in other examples, the inlets 1102 and outlets 1104 for each chamber are on different sides. Furthermore, the inlets 1102 and outlets 1104 can be on either side, depending on the flow path in which the balancing pod is integrated.

In one example, balancing of flow in the internal dialysate circuit works as follow. A set of pneumatically operated valves 211, 212, 213, 241, 242 has its operation synchronized and controlled together, where valves 211, 212, 213 are ganged and valves 241 and 242 are ganged, and a second set of pneumatically operated valves 221, 222, 223, 231, 232 similarly have its operation synchronized and controlled together, where valves 221, 222, 223 are ganged, and valves 231 and 232 are ganged. At a first point of time, the first set of valves 211, 212, 213, 241, 242 is opened while the second set of valves 221, 222, 223, 231, 232 is closed. Fresh dialysate flows into balancing chamber 341 while used dialysate flows from dialyzer 14 into pod pump 161. Fresh dialysate does not flow into balancing chamber 342 since valve 221 is closed. As fresh dialysate flows into balancing chamber 341, used dialysate within balancing chamber 341 is forced out and exits balancing circuit 143 (the used dialysate cannot enter pod pump 161 since valve 223 is closed). Simultaneously, pod pump 162 forces used dialysate present within the pod pump into balancing chamber 342 (through valve 213, which is open; valves 242 and 222 are closed, ensuring that the used dialysate flows into balancing chamber 342). This causes fresh dialysate contained within balancing chamber 342 to exit the balancing circuit 143 into dialyzer 14. Also, pod pump 161 draws in used dialysate from dialyzer 14 into pod pump 161.

Once pod pump 161 and balancing chamber 341 have filled with dialysate, the first set of valves 211, 212, 213, 241, 242 is closed and the second set of valves 221, 222, 223, 231, 232 is opened. Fresh dialysate flows into balancing chamber 342 instead of balancing chamber 341, as valve 212 is closed while valve 221 is now open. As fresh dialysate flows into balancing chamber 342, used dialysate within the chamber is forced out and exits balancing circuit, since valve 213 is now closed. Also, pod pump 162 now draws used dialysate from the dialyzer into the pod pump, while used dialysate is prevented from flowing into pod pump 161 as valve 232 is now closed and valve 222 is now open. Pod pump 161 forces used dialysate contained within the pod pump (from the previous step) into balancing chamber 341, since valves 232 and 211 are closed and valve 223 is open. This causes fresh dialysate container within balancing chamber 341 to be directed into the dialyzer 14 (since valve 241 is now open while valve 212 is now closed). At the end of this step, pod pump 162 and balancing chamber 342 have filled with dialysate. This puts the state of the system back into the configuration at the beginning of this description, and the cycle is thus able to repeat, ensuring a constant flow of dialysate to and from the dialyzer 14. In one example, the fluid (e.g. pneumatic) pressures on the control side of the balancing chamber valves are monitored to ensure they are functioning (e.g., opening and closing) properly.

As a specific example, a vacuum (e.g., 4 p.s.i. of vacuum) can be applied to the port for the first set of valves, causing those valves to open, while positive pressure (e.g., 20 p.s.i. of air pressure) is applied to the second set of valves, causing those valves to close (or vice versa). The pod pumps each urge dialysate into one of the volumes in one of the balancing chambers 341, 342. By forcing dialysate into a volume of a balancing chamber, an equal amount of dialysate is squeezed by the diaphragm out of the other volume in the balancing chamber. In each balancing chamber, one volume is occupied by fresh dialysate heading towards the dialyzer and the other volume is occupied by used dialysate heading from the dialyzer. Thus, the volumes of dialysate entering and leaving the dialyzer are kept substantially equal.

The bypass pump 35 can direct the flow of dialysate from the dialyzer 14 through balancing circuit 143. without passing through either of pod pumps 161 or 162. In this examples, the bypass pump 35 is a pod pump, similar to those described above, with a rigid chamber and a flexible diaphragm dividing each chamber into a fluid compartment and a control compartment. This pump may be the same or different from the other pod pumps and/or metering pumps described above. When control fluid is used to actuate the bypass pump 35, the additional drop in pressure on the exiting (spent) dialysate side of the dialyzer causes additional ultrafiltration of fluid from the blood in the dialyzer. This may cause a net efflux of fluid from the patient's blood, through the dialyzer, and ultimately to drain. Such a bypass may be useful, for example, in reducing the amount of fluid a patient has, which is often increased due to the patient's inability to excrete excess fluid (primarily water) through the kidneys. As shown in FIG. 4, the bypass pump 35 may be controlled by a control fluid (e.g., air), irrespective of the operation of pod pumps 161 and 162. This configuration may allow for easier control of net fluid removal from a patient, without having to operate the inside dialysate pumps either out of balance or out of phase with the blood pumps in order to achieve such fluid withdrawal from the patient.

To achieve balanced flow across the dialyzer, the blood flow pump, the pumps of the balancing circuit, and the pumps of the directing circuit (discussed below) may be operated to work together to ensure that flow into the dialyzer is generally equal to flow out of the dialyzer. If ultrafiltration is required, the ultrafiltration pump (if one is present) may be run independently of some or all of the other blood and/or dialysate pumps to achieve the desired ultrafiltration rate.

To prevent outgassing of the dialysate, the pumps of the balancing circuit may be kept at pressures above atmospheric pressure. In contrast, however, the blood flow pump and the directing circuit pumps use pressures below atmosphere to pull the diaphragm towards the chamber wall to complete a fill stroke. Because of the potential of fluid transfer across the semi-permeable membrane of the dialyzer and because the pumps of the balancing circuit run at positive pressures, the balancing circuit pumps may be able to use information from the blood flow pump(s) in order to synchronize the delivery strokes of the balancing circuit chambers to the dialyzer with the delivery strokes of the blood pump.

When running in such a balanced mode, if there is no delivery pressure from the blood flow pump, the balancing circuit pump diaphragm will push fluid across the dialyzer into the blood and the alternate pod of the balancing circuit will not completely fill. For this reason, in preferred examples, the blood flow pump reports when it is actively delivering a stroke. When the blood flow pump is delivering a stroke the inside dialysate pump operates. When the blood flow pump is not delivering blood, the valves that control the flow from the dialyzer to the inside dialysate pumps (and other balancing valves ganged together with these valves, as previously discussed) may be closed to prevent any fluid transfer from occurring from the dialysate side to the blood side. During the time the blood flow pump is not delivering, the inside dialysate pumps are effectively frozen, and the inside dialysate pump delivery stroke resumes once the blood flow pump starts delivering again. The inside dialysate pump fill pressure can be set to a minimal positive value to ensure that the pump operates above atmosphere at minimal impendance. Also, the inside dialysate pump delivery pressure can be set to the blood flow pump pressure to generally match pressures on either side of the dialyzer, minimizing flow across the dialyzer during delivery strokes of the inside dialysate pump.

In another example, the aside dialysate pump delivers dialysate to the dialyzers at a pressure slightly above the pressure at which blood is delivered to the dialyzer. This ensures that a full balance chamber of clean dialysate gets delivered to the dialyzer. On the return side, the inside dialysate pump can fill with spent dialysate from the dialyzer at a slightly lower pressure than the outlet pressure on the blood side of the dialyzer, ensuring that the receiving dialysate pump chamber can fill. This in turn ensures that there is enough dialysate available to complete a full stroke in the balancing chamber. Flows across the semipermeable membrane caused by these differential, pressures will tend to cancel each other; and the pumping algorithm otherwise attempts to match the average pressures on the dialysate and blood sides of the dialyzer.

It is generally beneficial to keep the blood flow as continuous as possible during therapy, as stagnant blood flow can result in blood clots. In addition, when the delivery flow rate on the blood flow pump is discontinuous, the balancing pump may pause its stroke more frequently, which can result in discontinuous and/or low dialysate flow rates. However, the flow through the blood flow pump can be discontinuous for various reasons. For instance, pressure may be limited within the blood flow pump, e.g., to +600 mmHg and/or -350 mmHg to provide safe pumping pressures for the patient. For instance, during dual needle flow, the two pod pumps of the blood flow pump can be programmed to run 180° out of phase with one another. If there were no limits on pressure, this phasing could always be achieved However to provide safe blood flow for the patient these pressures are limited. If the impedance is high on the fill stroke (due to a small needle, very viscous blood, poor patient access, etc.), the negative pressure limit may be reached and the fill flow rate will be slower then the desired fill flow rate. Thus the delivery stroke must wait for the previous fill stroke to finish, resulting in a pause in the delivery flow rate of the blood flow pump. Similarly, during single needle flow, the blood flow pump may be run at 0[deg.] phase, where the two blood flow pump pod pumps are simultaneously emptied and filled. When both pod pumps are filled, the volumes of the two pod pumps are delivered. In an example, the sequence of activation causes a first pod pump and then a second pod pump to fill, followed by the first pod pump emptying and then the second pod pump emptying. Thus the flow in single needle or single lumen arrangement may be discontinuous.

One method to control the pressure saturation limits would be to limit the desired flow rate to the slowest of the fill and deliver strokes. Although this would result in slower blood delivery flow rates, the flow rate would still be known and would be more continuous, which would allow for more accurate and continuous dialysate flow rates. Another method to make the blood flow rate more continuous in single needle operation would be to use maximum pressures to fill the pods so the fill time would be minimized. The desized deliver time could then be set to be the total desired stroke time minus the time that the fill stroke took. However, the less continuous the blood flows, the more the dialysate flow rate may have to be adjusted upward during blood delivery to the dialyzer to make up for the time that the dialysate pump is stopped when the blood flow pump is filling. If this is done with the correct timing, an average dialysate flow rate taken over several strokes can still match the desired dialysate flow rate.

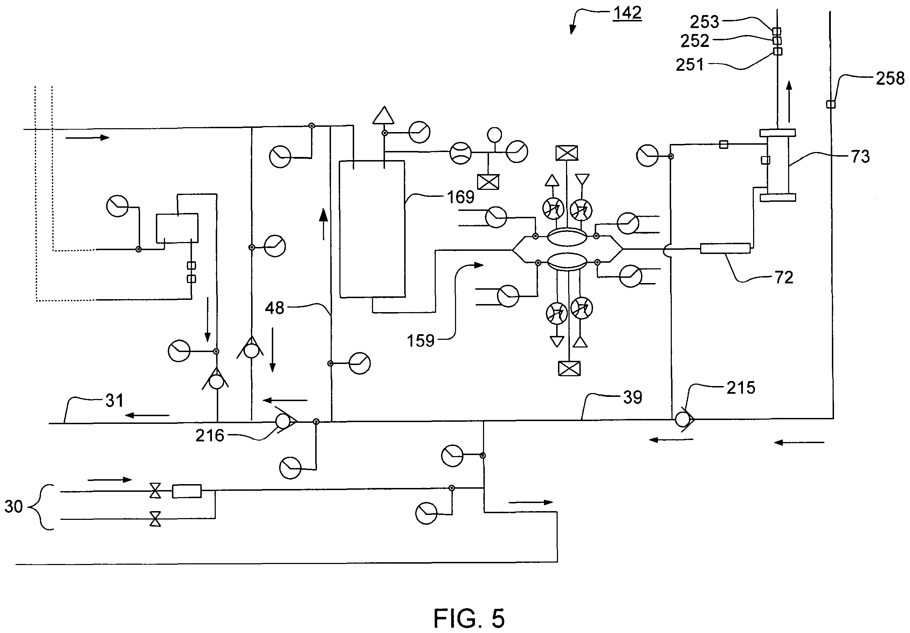

FIG. 5 shows a close up of the directing circuit 142 in the FIG. 2 dialysis system. In this example, the directing circuit 142 can provide dialysate from a dialysate tank 169 via a dialysate pump 159 to a heater 72 and the ultrafilter 73. The heater 72 may be used to warm the dialysate to body temperature, and/or a temperature such that the blood in the blood flow circuit is heated by the dialysate, and the blood returning to the patient is at body temperature or higher. In some cases, the heater 72 may be connected to a control system such that dialysate that is incorrectly heated (i.e., the dialysate is too hot or too cold) may be recycled (e.g., back to the dialysate tank 169) or sent to drain instead of being passed to the dialyzer. The heater 72 may also be used, in some cases, for disinfection or sterilization purposes. For instance, water may be passed through the hemodialysis system and heated using the heater such that the water is heated to a temperature able to cause disinfection or sterilization to occur, e.g., temperature of at least about 70°C, at least about 80°C, at least about 90°C, at least about 100°C, at least about 110°C, etc.

The flow of dialysate through the directing circuit 142 may be controlled (at least in part) by operation of the dialysate pump 159. In addition, the dialysate pump 159 may control flow through the balancing circuit 143. For instance, as discussed above, fresh dialysate from the directing circuit 142 flows into balancing chambers 341 and 342 of balancing circuit. 143. The dialysate pump 159 may be used as a driving force to cause the fresh dialysate to flow into these balancing chambers. In one set of examples, dialysate pump 159 includes a pod pump, e.g., similar to those described above.

The dialysate may also be faltered to removed contaminants, infectious organisms, pathogens, pyrogens, debris, and the like, for instance, using an ultrafilter 73. The ultrafilter 73 may be positioned in any suitable location in the dialysate flow path, for instance, between the directing circuit and the balancing circuit, e.g., as shown, and/or the ultrafilter 73 may be incorporated into the directing circuit or the balancing circuit. If an ultrafilter is used, its pore size may be chosen to prevent species such as these from passing through the filter.

In some cases, the ultrafilter 73 may be operated such that waste from the filter (e.g., the retentate stream) is passed to a waste stream, such as waste line 39 in FIG. 5. In some cases, the amount of dialysate flowing into the retentate stream may be controlled. For instance, if the retentate is too cold (i.e., heater 72 is not working, or heater 72 is not heating the dialysate to a sufficient temperature, the entire dialysate stream (or at least a portion of the dialysate) may be diverted to waste line 39, and optionally, recycled to dialysate tank 169 using line 48. Flow from the filter 73 may also be monitored for several reasons, e.g., using temperature sensors (e.g., sensors 251 and 252), conductivity sensors (for confirming dialysate concentration, e.g., sensor 253), or the like.

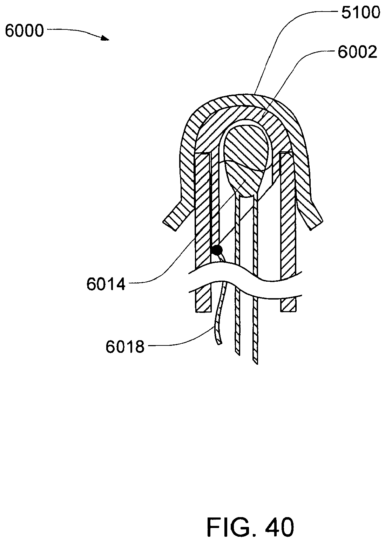

Referring now to FIG. 40, a sensing probe 6000 and a thermal well 5100 are shown coupled and outside of a fluid line. The thermal well 5100 can be in a fluid line, a protective sleeve, any disposable, machine chamber, cassette or container. However, for purposes of this description of the illustrated example, the thermal well 5100 is taken to be anywhere where it is used to determine thermal and/or conductive properties of a subject media.

A subject media is in contact with the outside of zone 5402 of the thermal well 5100. Thermal energy is transferred from the subject media to the thermal well 5100 and further transferred to the tip 6002 of the sensing probe 6000. Thermal energy is then conducted to the thermal sensor 6014. The thermal sensor 6014 communicates via leads 6016 with equipment that can determine the temperature of the subject media based on feedback of the thermal sensor 6014. In examples where conductivity sensing is also desired, lead 6018 communicates with equipment that can determine the conductivity of the subject media. With respect to determining the conductivity of the subject media, in addition to the lead 6018, a second electrical lead/contact (not shown) would also be used. The second lead could be a second sensor apparatus as shown in FIG. 40, or, alternatively, a second probe that is not necessarily the same as the sensor apparatus shown in FIG. 40, but rather, any probe or apparatus capable of sensing capacitance of the subject media, including, an electrical contact.

The ultrafilter and the dialyzer may provide redundant screening methods for the removal of contaminants, infectious organisms, pathogens, pyrogens, debris, and the like. Accordingly, any contaminant would have to pass through both the ultrafilter and the dialyzer before reaching a patient's blood. Even in the event that either the ultrafilter or dialyzer integrity fails, the other may still be able to maintain dialysate sterility and prevent contaminants from reaching the patient's blood.

The directing circuit 142 may also be able to route used dialysate coming from a balancing circuit to a drain, e.g., through waste line 39 to drain 31. The drain may be, for example, a municipal drain or a separate container for containing the waste (e.g., used dialysate) to be properly disposed of. In some cases, one or more check or "one-way" valves (e,g., check valves 215 and 216) may be used to control flow of waste from the directing circuit 142 and from the system 5. Also, in certain instances, a blood leak sensor (e.g., sensor 258) may be used to determine if blood is leaking through the dialyzer 14 into the dialysate flow path. In addition, a liquid sensor can be positioned in a collection pan at the bottom of the hemodialysis unit to indicate leakage of either blood or dialysate, or both, from any of the fluid circuits.

The directing circuit 142 may receive water from a water supply 30, e.g., from a container of water such as a bag, and/or from a device able to produce water, e.g., a reverse osmosis device. In some cases, the water entering the system is set at a certain purity, e.g., having ion concentrations below certain values, The water entering into the directing circuit 142 may be passed on to various locations, e.g., to a mixing circuit 25 for producing flesh dialysate and/or to waste line 39. In some cases, valves to the drain 31 and various recycle lines are opened, and conduits 67 may be connected between directing circuit 142 and blood flow circuit 141, such that water is able to flow continuously around the system. If heater 72 is also activated, the water passing through the system will be continuously heated, e.g., to a temperature sufficient to disinfect the system.

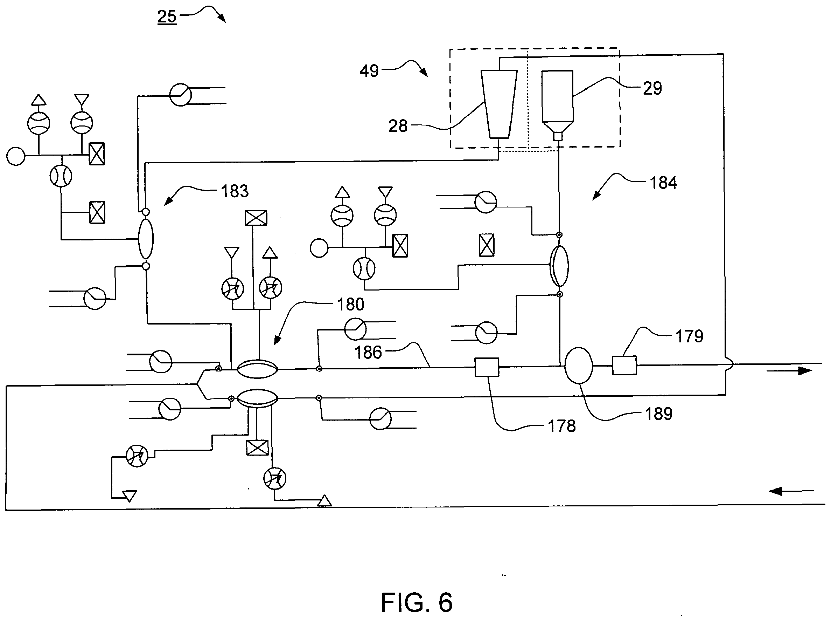

FIG. 6 shows a close-up view of the mixing circuit 25 in the illustrative dialysis system of FIG. 2. Water from the directing circuit 142 flows into the mixing circuit 25 due to action of a pump 180. In this system, the pump 180 includes one or more pod pumps, similar to those described above. In some cases, a portion of the water is directed to reagent ingredients 49, e.g., for use in transporting the ingredients, such as the bicarbonate 28, through the mixing circuit 25. In some cases, sodium chloride and/or the sodium bicarbonate 28 may be provided in a powdered or granular form, which is mixed with water provided by the pump 180. Bicarbonate from bicarbonate source 28 is delivered via bicarbonate pump 183 to a mixing line 186, which also receives water from the directing circuit 142. Acid from an acid source 29 (which may be in a liquid form) is also pumped via an acid pump 184 to the mixing line 186. The ingredients 49 (water, bicarbonate, acid, NaCl, etc.) are mixed in mixing chamber 189 to produce dialysate, which then flows out of mixing circuit 25 to the directing circuit 142. Conductivity sensors 178 and 179 are positioned along mixing line 186 to ensure that as each ingredient is added to the mixing line, it is added at proper concentrations. The volumes delivered by the water pump 180 and/or the other pumps may be directly related to the conductivity measurements, so the volumetric measurements may be used as a cross-check on the composition of the dialysate that is produced. This may ensure that the dialysate composition remains safe even if a conductivity measurement becomes inaccurate during a therapy.

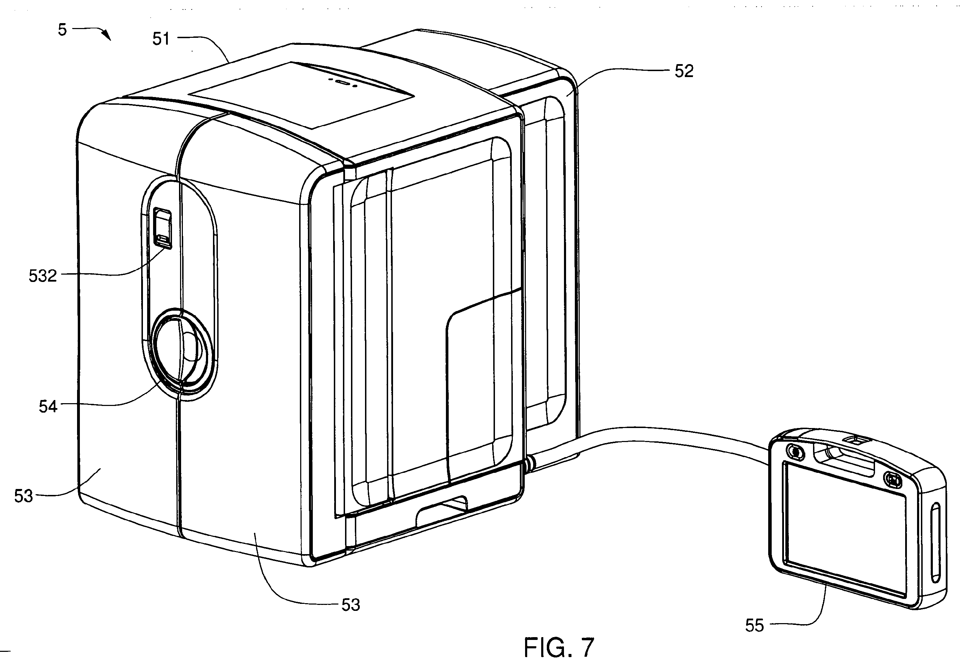

FIG. 7 shows a perspective view of a hemodialysis system 5 that incorporates various examples from the present teaching. In this regard, the system 5 includes a dialysis unit 51 and a power unit module 52 that are shown joined together. In this illustrated system, the dialysis unit 51 has a housing that contains suitable components for performing hemodialysis, such as a dialyzer, one or more pumps to circulate blood through the dialyzer, a source of dialysate, and one or more pumps to circulate the dialysate through the dialyzer. For example, the dialysis unit 51 may include the mixing circuit 25, blood flow circuit 141, the balancing circuit 143 and the directing circuit 142 as described above. The dialysis unit 51 may also include all blood circuit connections and dialysate fluidic connections needed for operation of the system 5. Patient access and other connections may be revealed by opening side-by-side vertical doors 53 via a handle 54 at a front side of the dialysis unit 51 housing. In this system, the dialysis unit 51 includes a control interface 55 (attached to the housing by a flexible cable in this example) that a user may use to control operation of the dialysis unit 51. The control interface 55 may include a display screes with a touch sensitive overlay to allow touch control and interaction with a graphical user interface presented on the screen. The control interface 55 may also include other features, such as push buttons, a speaker, a microphone for receiving voice commands, a digital camera, and so on. The back side of the control interface 55 may include a retractable "kick-stand" (not shown) that allows the control interface 55 to be positioned on top of the dialysis unit 51 housing. Deploying the retractable "kick-stand" permits the control interface 55 to be placed in a near- vertical position to allow proper viewing of the display screes.

The power unit 52 housing may contain suitable components for providing operating power to the dialysis unit 51, e.g., pneumatic pressure/vacuum to power the pumps, valves and other components of the dialysis unit 51. "Pneumatic," as used herein, means using air or other gas to move a flexible diaphragm or other member. (It should be noted that air is used by way of example only, and other control fluids, such as nitrogen ($N_2$), $CO_2$, water, an oil, etc., may be used instead). As discussed above, the pumps and valves of the dialysis unit 51 may operate on pneumatic power, and thus the power unit 52 may provide one or more pneumatic sources for use by the dialysis unit 51. In this way, the dialysis unit 51 need not necessarily be arranged to generate and/or store the necessary pneumatic power needed, but instead may rely on the power unit module 52. The power unit 52 may include one or more pneumatic pumps to generate desired air pressure and/or vacuum, one or more accumulators or other devices to store pneumatic power, valves, conduits and/or other devices to control flow of pneumatic power in the power unit 52, as well as a controller having suitable components, such as a programmed general purpose data processor, memory, sensors (e.g., to detect pressure, temperature, etc.), relays, actuators, and so on.

In one system, the pneumatic power (e.g., air under suitable pressure/vacuum) may be supplied by the power unit 52 to the dialysis unit 51 via one or more supply tanks or other pressure sources. For instance, if two tanks are used in the power unit 52, one supply tank may be a positive pressure reservoir, may have a set point of 750 mmHg (gauge pressure) (1 mmHg is about 133.3 pascals). The other supply tank can be a vacuum or negative pressure reservoir, and may have a set point of -450 mmHg (gauge pressure). This pressure difference may be used, for instance, between the supply tanks and the required pod pump pressure to allow for accurate control of the variable valves to the pod pumps. The supply pressure limits can be set based on maximum pressures that can be set for the patient blood flow pump plus some margin to provide enough of a pressure difference for control of the variable valves. Thus, in some cases, the two tanks may be used to supply pressures and control fluids for all of the dialysis unit 51 functions.

In one system, the power unit 52 may include two independent compressors to service the supply tanks. Pressure in the tanks can be controlled using any suitable technique, for instance, with a simple "bang-bang" controller (a controller that exists in two states, i.e., in an on or open state, and an off or closed state), or with more sophisticated control mechanisms, depending on the system. As an example of a bang-bang controller, for the positive tank, if the actual pressure is less than a set point, the compressor servicing the positive tank is turned on. If the actual pressure is greater than a set point, the compressor servicing the positive tank is turned off. The same logic may be applied to the vacuum tank and control of the vacuum compressor with the exception that the sign of the set point term is reversed. If the pressure tanks are not being regulated, the compressor is turned off and the valves are closed.

Tighter control of the pressure tanks can be achieved by reducing the size of the hysteresis band, however this may result in higher cycling frequencies of the compressor. If very tight control of these reservoirs is required, the bang-bang controller could be replaced with a proportional-integral-derivative ("PID") controller and using pulse width modulation ("PWM") signals on the compressors. Other methods of control are also possible.

Other pressure sources may be used in other examples, and in some cases, more than one positive pressure source and/or more than one negative pressure source may be used. For instance, more than one positive pressure source may be used that provides different positive pressures (e.g., 1000 mmHg and 700 mmHg), which may be used to minimize leakage. For example, high positive pressure can be used to control valves, whereas lower positive pressures can be used to control pumps. This limits the amount of pressure that can potentially be sent to the dialyzer or to the patient, and helps to keep actuation of the pumps from overcoming the pressures applied to adjacent valves. A non-limiting example of a negative pressure is -400 mmHg. In some cases, the negative pressure source may be a vacuum pump, while the positive pressure pump may be an air compressor.

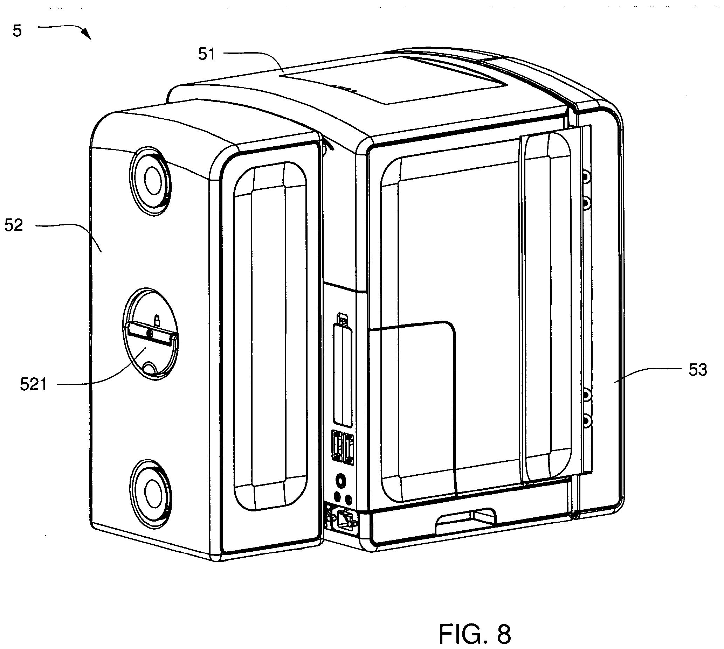

Moreover, the power unit 52 may be selectively connectable to the dialysis unit 51, e.g., to allow different power units 52 to be interchanged. For example, the dialysis unit 51 may be arranged to work with different types of power units 52, such as power units 52 that use electrical power to generate the pneumatic power supply, as well as power units 52 that use stored pneumatic power (e.g., pressurized air stored in one or more high pressure tanks). Thus, a power unit 52 may be interchanged for another unit 52, in case of failure or other requirements. For example, it may be desired to use the system 5 in an area where noise generation is unacceptable, such as when nearby people are sleeping. In this case, it may be desirable to use a power unit 52 that uses stored pneumatic power, rather than a unit 52 that generates pneumatic power by running pumps or other noise generating equipment. As shown in FIG. 8, the power unit 52 may be disconnected from the dialysis unit 51 by manipulating a handle 521. For example, turning the handle 521 may unlock the power unit 52 from the dialysis unit 51, disengaging not only mechanical connections between the housings, but also power and/or communications connections between the two. An interface (not shown) between the dialysis unit 51 and the power unit 52 may permit the units to exchange pneumatic power (from the power unit 52 to the dialysis unit 51) as well as electrical power, control communications, and other. The dialysis unit 51 may have connection points for electrical power (e.g., standard 115V, 15amp power found in most home power outlets), external communication (such as Ethernet, or any other suitable connection suitable for communication), a water supply, and so on. The dialysis unit 51 may provide electrical power or other connections to the power unit 52, if desired.

The dialysis unit 51 may include a controller to control flow of control fluid for various components of the system 5, as well as perform other desired functions. In some cases, the control fluid may be held at different pressures within the various tubes or conduits. For instance, some of the control fluid may be held at positive pressure (i.e., greater than atmospheric pressure), while some of the control fluid may be held at negative pressures (less than atmospheric pressure). In addition, in certain examples, the controller may have components that are kept separate from the various liquid circuits. This configuration has a number of advantages. For example, the liquid circuits in the dialysis unit 51 may be heated to disinfection temperatures and/or exposed to relatively high temperatures or other harsh conditions (e.g., radiation) to effect disinfection, while electronic components of the controller may not be exposed to such harsh conditions, and may even be kept separate by an insulating wall (e.g., a "firewall") or the like. That is, the dialysis unit housing may have two or more compartments, e.g., one compartment with electronic and other components that may be sensitive to heat or other conditions, and another compartment with liquid circuit components that are heated or otherwise treated for disinfection.

Thus, the system 5 may include a "cold" section (which is not heated), and a "hot" section, portions of which may be heated, e.g., for disinfection purposes. The cold section may be insulated from the hot section through insulation. In one system, the insulation may be molded foam insulation, but in other systems can be any type of insulation, including but not limited to a spray insulation, an air space, insulation cut from sheets, etc. In one system, the cold section includes a circulation system, e.g., a fan and/or a grid to allow air to flow in and out of the cold box. In some cases, the insulation may be extended to cover access points to the "hot" section, e.g., doors, ports, gaskets, and the like. For instance, when the "hot" section is sealed, the insulation may completely surround the "hot" section in some cases.

Non-limiting examples of components that may be present within the "cold" section include power supplies, electronic, power cables, pneumatic controls, or the like. In some cases, at least some of the fluids going to and from the "hot" section may pass through the "could" section; however, in other cases, the fluids may pass to the "hot" section without passing through the "cold" section.

Non-limiting examples of components that may be present within the "hot" section include cassettes (if present), fluid lines, temperature and conductivity sensors, blood leak sensors, heaters, other sensors, switches, emergency lights, or the like. In some cases, some electrical components may also be included in the "hot" section. These include, but are not limited to, a heater. In one system, the heater can be used to heat the hot box itself, in addition to fluid. In some systems, the heater 72 heats the entire "hot" section to reach a desired temperature.

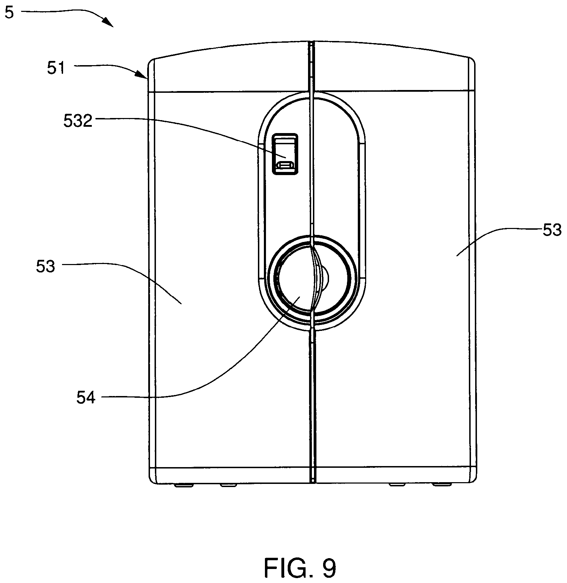

The dialysis unit 51 housing may include vertical side-by-side doors that can be opened to expose all mechanical interface points for blood flow circuitry and connections for dialysate circuitry, i.e., all connection points for patient blood connections and acid/bicarbonate connections, that must be made by a user to use the dialysis unit 51. FIG. 9 shows a front view of the dialysis unit 51 with the vertical side-by-side doors 53 in a closed state. In this arrangement, the doors 53 may block access to connection points for patient blood connections and acid/bicarbonate connections as well as seal the interior of the unit housing so as to allow heat retention suitable for disinfection. The seal provided by the doors 53 may be hermetic, preventing or substantially resisting any air exchange between the housing interior and an exterior environment, or may be of a somewhat lesser quality yet still allow for disinfection.

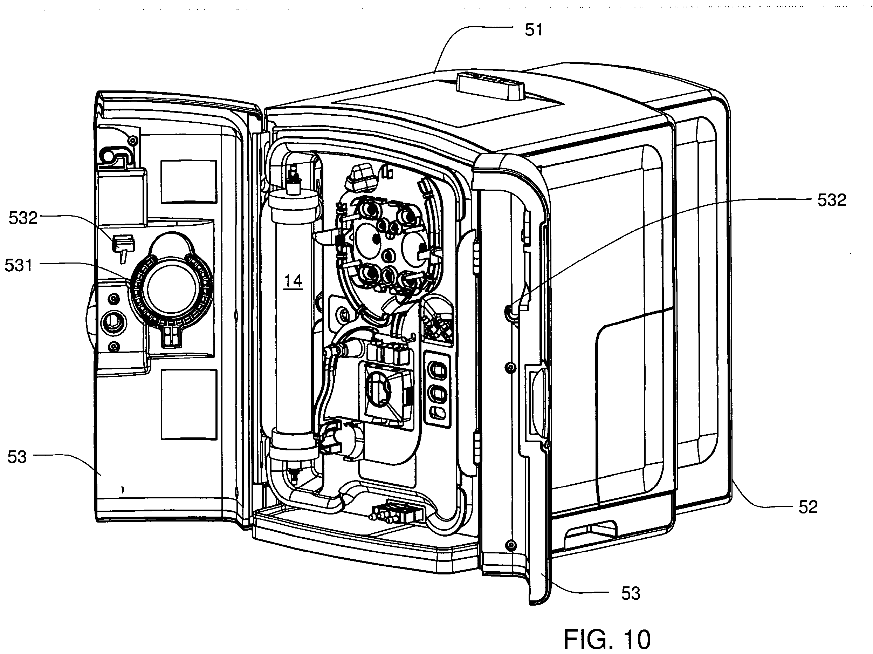

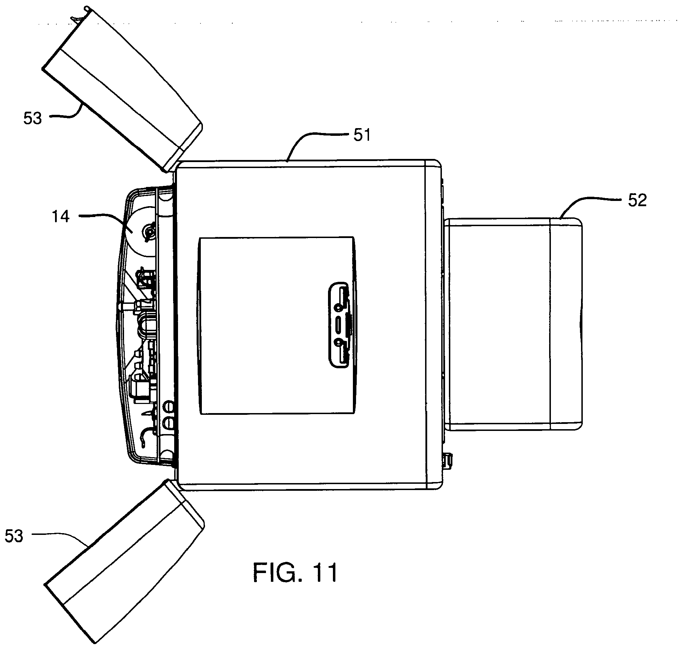

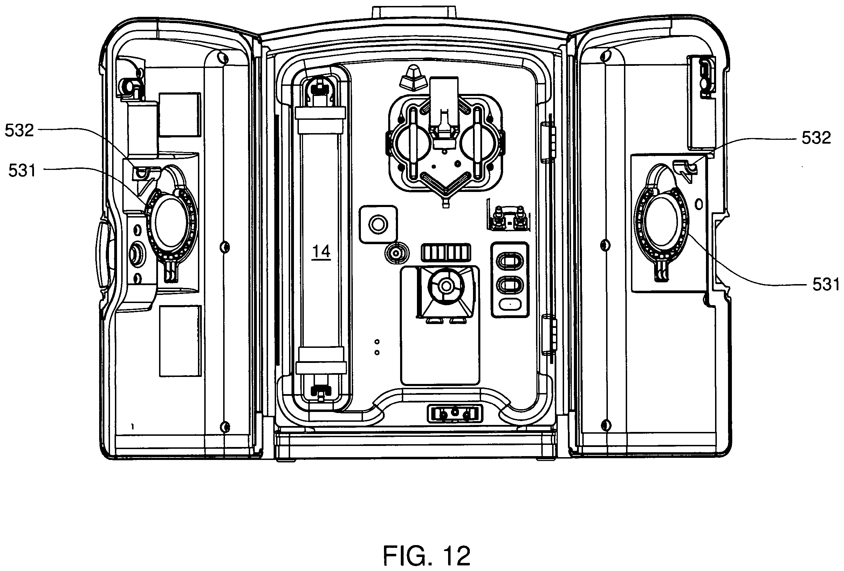

In this example, the doors 53 are connected to the dialysis unit 51 housing by a dual hinge arrangement such that the doors 53 can be opened to two different states of opening. FIGs. 10-13 show the doors 53 in a first state of opening. In this state, the doors 53 expose all user-made connections for the blood circuit connections and for the dialyzer circuitry, including the dialyzer 14 itself and for reagent materials, such as consumable acid/bicarbonate materials. This position also exposes several other features, such as holders 531 for an acid/bicarbonate container (not shown) and hooks 532 that may be used to hold any suitable item, such as the control interface 55, which may be hung by its handle on one of the hooks 532. (See also FIG. 7 which shows a hook 532 on the front of the left door 53 which may be folded out to receive the control interface 55 or other item.) The holders 531 may be folded down from their position shown in the figures (i.e., folded up and into recesses in the doors 53) so as to extend horizontally from the doors 53. The holders 531 have a "C" shaped receiving section to receive and hold an acid/bicarbonate container, but of course could be shaped or otherwise arranged in any suitable way.

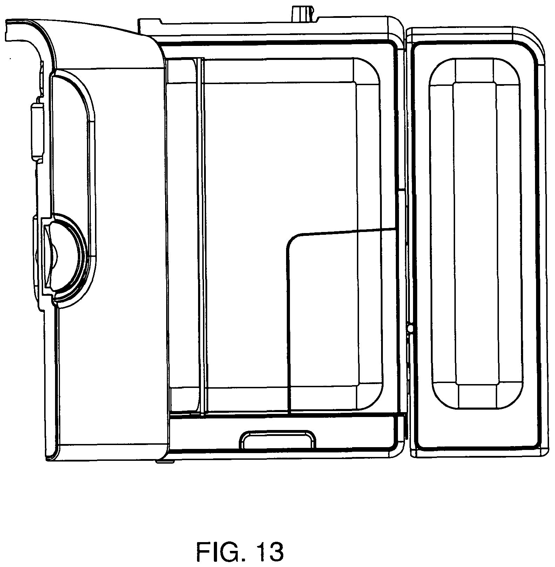

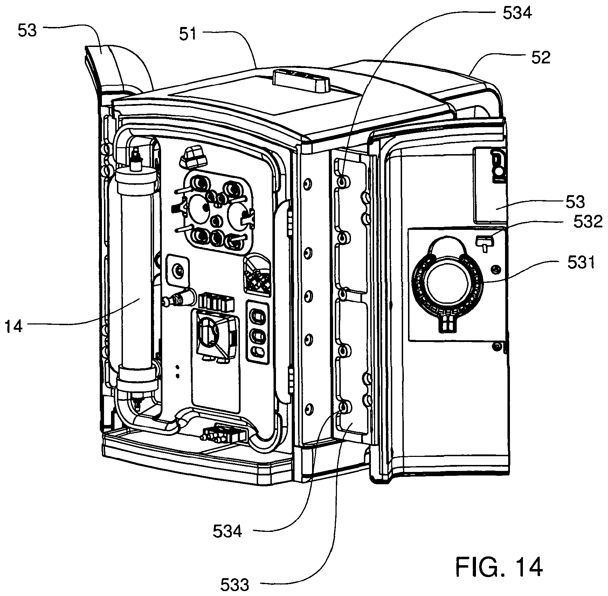

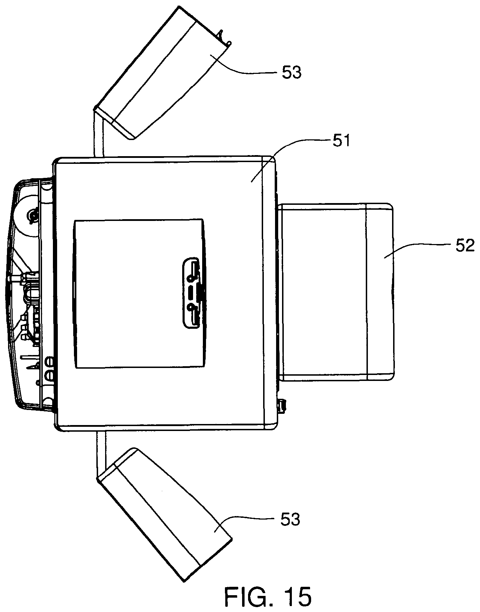

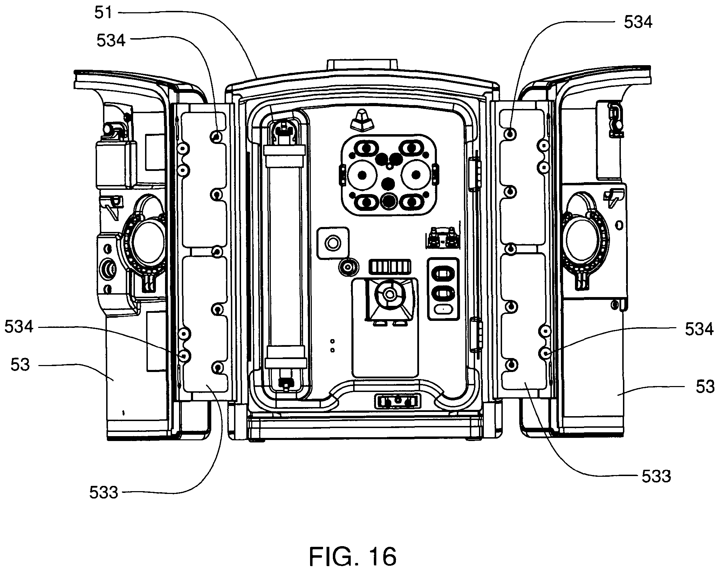

FIGs. 14-16 show the doors 53 in a second state of opening in which a hinge plate 533 for each door 53 is pivoted outward and away from the dialysis unit housing 51. The hinge plates 533, which in this example extend vertically along almost the entire height of the dialysis unit housing 51, are pivotally attached to the doors 53 at a first, outer end, and are pivotally attached at a second inner end to the dialysis unit housing 51. (Of course, it should be understood that the hinge plates 533 could be arranged and/or positioned differently, e.g., at the top and bottom of the doors 53 as is found in many refrigerator door arrangements, each plate 533 may include two or more portions that are vertically separated from each other, etc.) Magnets 534 attached to the hinge plates 533 may interact with corresponding magnets (or other suitable components, such as a steel elements) attached to the dialysis unit housing 51 so as to attract the hinge plates 533 toward the dialysis unit housing 51, thus tending to keep the hinge plates 533 in the position shown in FIGs. 10-13. (Of course, the magnets 534 could be positioned on the unit housing, and the hinge plates 533 could have suitable elements (such as pieces of steel) that are attracted to the magnets 534.) The doors 53 in this example also include magnets attached near the hinge plates 533 so that when the doors 53 are opened to the first state as shown in FIGs 10-13, the magnets interact with corresponding magnets in the hinge plates 533 to help keep the doors 53 in an open position relative to the hinge plate 533. These magnets will also help maintain the relative position of the doors 53 and the hinge plates 533 when the hinge plates 533 are opened to the second state shown in FIGs. 13-16.

Although magnets are used in this illustrative embodiment as part of a retainer member to help the doors 53 and/or hinge plates 533 stay in a particular state of opening or closing, other arrangements for a retainer member are possible. For example, the hinge connection between the doors 53 and the hinge plates 533 and/or the connection between the hinge plates 533 and the housing 51 may include a detent arrangement that serves to resiliently hold the door 53 or hinge plate 533 in a particular position relative to the other part (the hinge plate or housing, respectively). In another examples, one or more springs may be used to help maintain the doors 53 in an open position relative to the hinge plates 533. In yet another example, the hinge plates 533 may have a friction or interference fit with a portion of the housing 51 that tends to maintain the hinge plates 533 in the closed position (adjacent the housing). Accordingly, a retainer member that functions to help maintain a door 53 in a particular position relative to its hinge plate 533, and/or that functions to help maintain a hinge plate 533 in a particular position relative to the housing 51, may take any one of a number of possible arrangements.

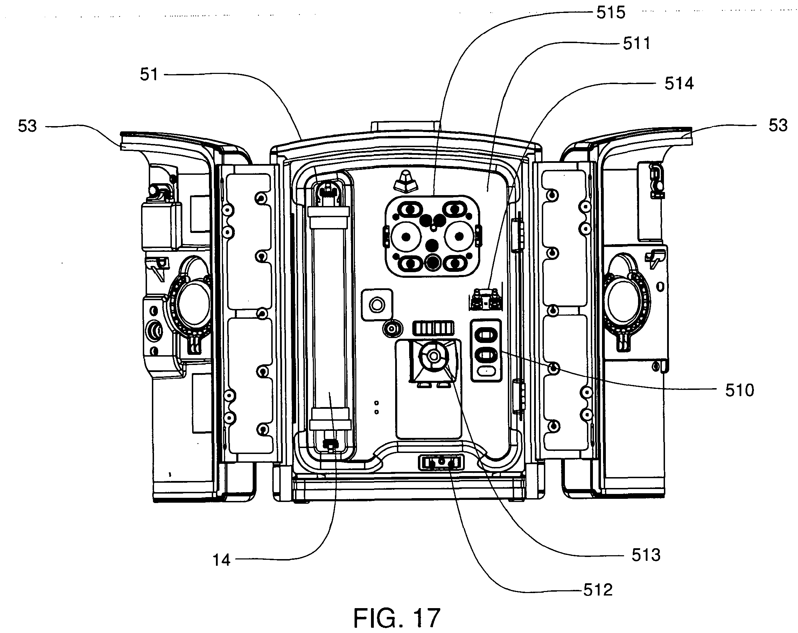

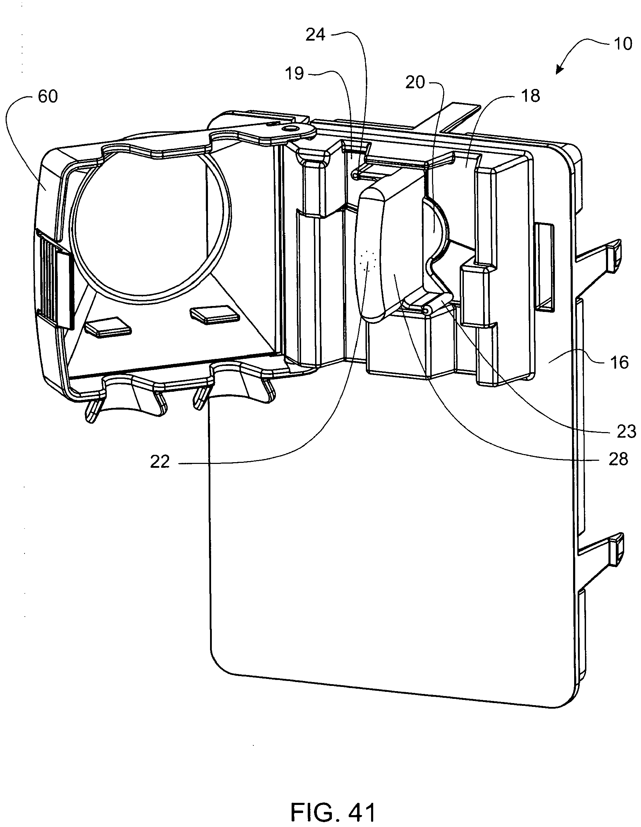

In certain examples, opening of the doors to the dialysis unit housing may reveal all of the user-made connections for blood circuit connections and dialysate fluidic connections needed for operation of the system 5. For example, as shown in FIG. 17, with the doors 53 in an open position (either the first for second state of opening) a front panel 511 of the dialysis unit 51 may be exposed. In this example, the front panel 51 1 carries several items or connection points that must be accessed by a user. For example, the dialyzers 14, which must be periodically replaced, is mounted to the front panel 511. The dialyzers 14 must be connected not only to the blood flow circuit 141, but also the balancing circuit 143. Also, a connection point 512 for an acid/bicarbonate source 49 is located at a lower end of the front panel 51 1. It is at this connection point 512 that a user may connect a source of consumable reagent ingredients 49 used by the dialysis unit 51 in making dialysate. An occluder 513 is also mounted on the front panel 511. The occluder 513 receives tubes of the blood flow circuit and controls the open/closed state of the tubes based on system operation. In short, the occluder 513 allows flow through the arterial and venous lines of the blood flow circuit unless there is a system problem, such as a leak, pump failure, overpressure situation, etc. In such case, the occluder 513 automatically closes the blood lines to prevent all flow to or from the patient. As shown in FIG. 41, occluder 10 includes a base 16 and a pathway 18 for receiving tubing. As shown in this figure, pathway 18 is an indentation within base 16 shaped for receiving tubing. However, in other examples, pathway 18 may be defined on base 16 in other ways, for example, by one or more ridges on the surface of base 16, by fasteners, loops, or rings on the surface of base 16, or the like. In addition, although pathway 18 is shown as being substantially straight in this figure, in other embodiments, pathway 18 may be curved in some fashion. In some cases, the pathway may be designed to be slightly smaller than the tubing it is designed to carry, thus requiring some force and/or deformation of the tubing in order for it to be properly positioned within the pathway.

The tubing may be bendable or flexible, for example, so that the tubing can be at least partially collapsed when occluding element 24 pushes into the tubing. For example, the tube may be formed from materials such as silicone, nylon, rubber, polyvinyl chloride, polyurethane, polyethylene, or the like. In some cases, the tubing is biocompatible. The tubing may be used, for example, to transport blood or other substances to or from a subject, e.g., as part of a dialysis machine or other medical infusion system.

Positioned on base 16 is an occluding member 20 that is rotationally moveable about a pivot 22. The occluding member may rotate completely around the pivot, or in some cases, the occluding member can rotate only partially around pivot 22. Pivot 22 may be immobilized with respect to base 16. For instance, pivot 22 may be a screw or a rod, or a similar structure, that is affixed within base 16. Occluding member 20 may be constructed such that when it is rotated around pivot 22, occluding element 24 can enter and at least partially obstruct pathway 18. Fluid flowing within tubing contained within pathway 18 may thus be partially or completely inhibited from flowing past occluding element 24. However, when occluding member 20 is in a second position, occluding element 24 does not enter into pathway 18, and thus fluid contained within a tubing in pathway 18 is not obstructed by occluding element 24.

As shown here, occluding member 20 may be in one of two positions. In some cases, however, the occluding member may be positioned in more than two positions. For instance, occluding member 20 may contain more that one occluding element, which may each enter into or at least partially obstruct pathway 18 and/or other pathways present within base 16, or occluding member 20 may be constructed to be positioned in different positions, some of which may cause occluding element 24 to be present within pathway 18 to various degrees, thereby causing occlusion of fluid flow to varying degrees. Occluding element 24 may have any shape suitable to at least partially block or obstruct flow of fluid within tubing contained within pathway 18 when occluding element 24 enters into or at least partially obstructs the pathway. As depicted in Fig. 41, occluding element 24 includes a semi-cylindrical shaped portion that enters pathway 18. However, other shapes may also be used, such as spheres, wedges, blocks, rods, or the like.

Also exposed on the front panel 511 are blood line connection points 514 for connecting the arterial and venous blood lines 203, 204 of the blood flow circuit 141 with the directing circuit 142 (as explained above with reference to FIGs. 2 and 3, the blood flow circuit 141 may be connected to the directing circuit 142). This connection is normally made at the end of treatment to allow the system to clean and disinfect the blood flow circuit 141. The front panel 511 also has a set of control ports 515 that mate with corresponding control ports on the blood pump portion of the blood flow circuit 141. The control ports 515 provide controlled levels of air pressure and/or vacuum to control the open/closed state of valves and to power the pumps of the blood flow circuit 141.

Also exposed on the front panel 511 is a user control panel 510. The user control panel 510 includes one or more buttons permitting the user to bypass the graphical user interface on control interface 55, providing an alternate method to control certain functions (e.g., critical functions) during hemodialysis. This may be important, for example, if the control interface 55 should ever fail during a dialysis treatment cession. Non-limiting examples of critical functions can include a "stop dialysis" or "pause dialysis" command and an "infuse dialysate solution" command.

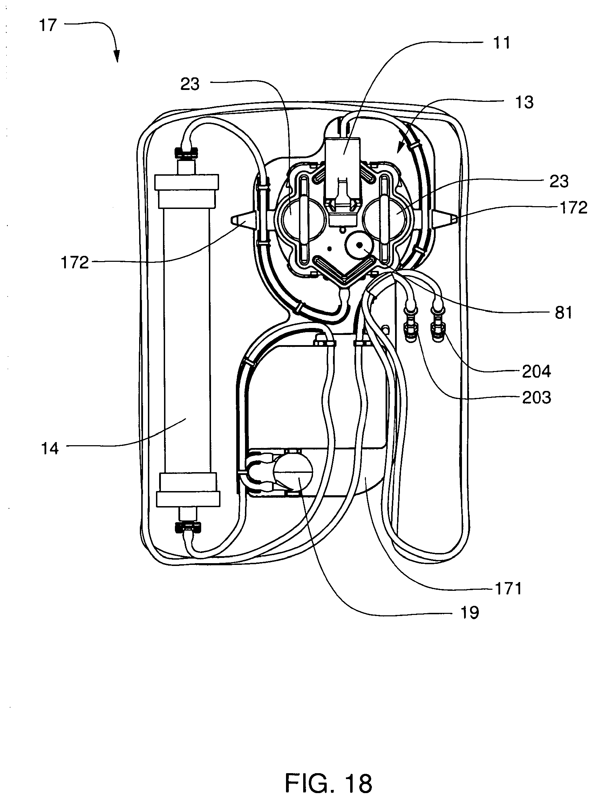

FIG. 17 does not show the arterial and venous lines 203, 204 for the blood flow circuit 141 because, in this example, the blood flow circuit 141 is formed as a blood circuit assembly that is removable from the front panel 511 of the dialysis unit 51, and the blood circuit assembly is not mounted on the front panel 511 in FIG. 17. FIG. 18 shows a front view of the blood circuit assembly 17, which is an embodiment of the present invention along with the dialyzer 14. The blood circuit assembly 17 includes various components discussed above, for example with reference to FIG. 3, that are mounted to a blood circuit organizing tray 171. The arterial and venous lines 203 and 204 (e.g., including lengths of flexible silicone tubing) are terminated with blood line connectors that may be arranged to provide a plug-in or press-in connection with the blood line connection points 514 as well as provide a screw-type connection used with standard patient access points (e.g., luer type patient access connectors). The arterial line 203 leads to an inlet at the top of the blood pump 13, which includes two pod pumps 23, valves and other components for controlling blood flow. Associated with the blood pump 13 are an air filter 81, an anticoagulant pump 80 (not shown), and an anticoagulant supply 11 (such as a vial of heparin).

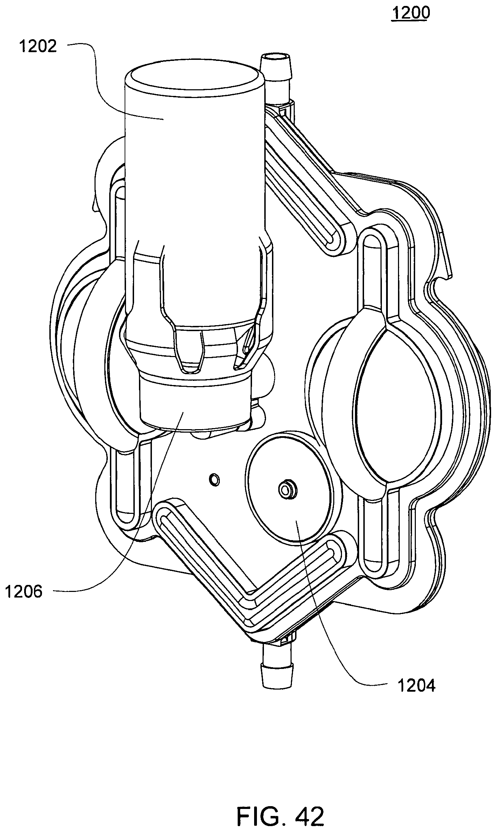

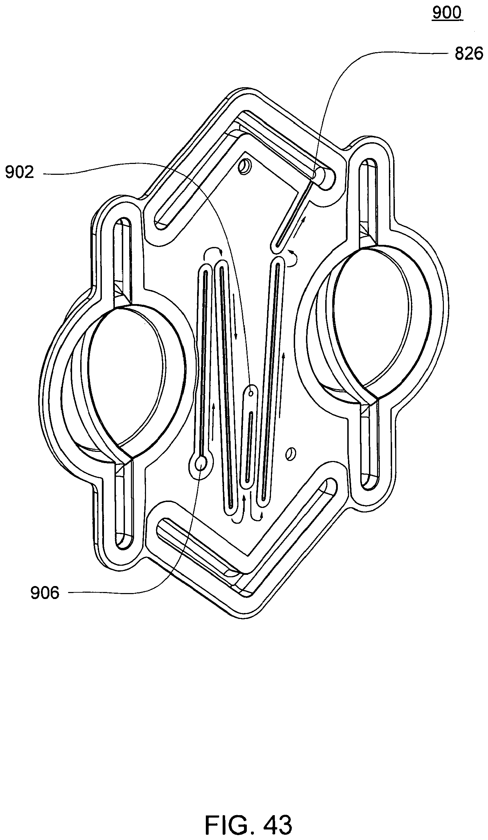

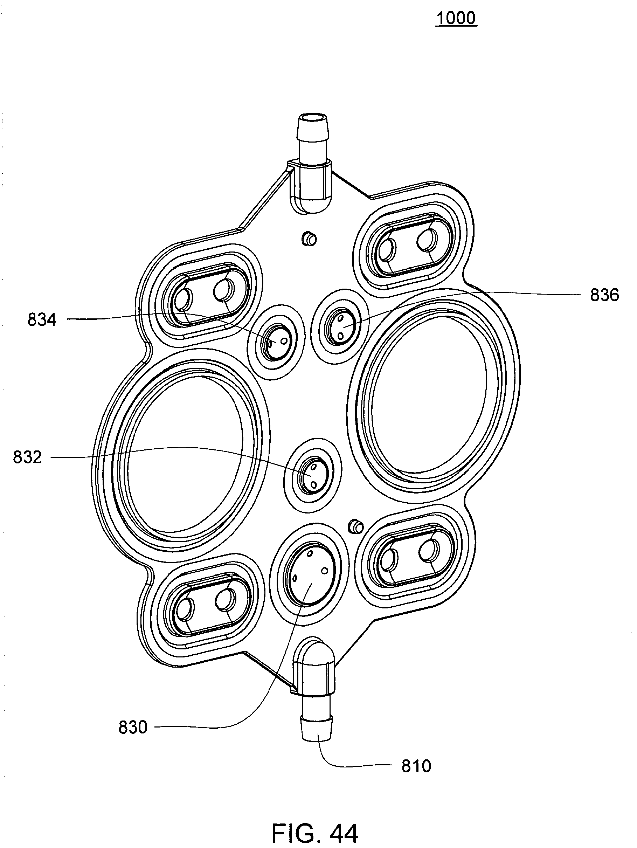

An exemplary blood pump cassette 1200 is shown in FIG. 42. A source container 1202 of medication such as heparin is placed top side down on container attachment 1206, which has a follow spike (not shown) piercing the stopper of the top of source container 1202. Air filter 1204 covers air vent 906, the inside portion 900 of which is shown in FIG. 43. FIG. 43 shows the metering pump fluid paths for source contained 1202 on the inside of the top cover of blood pump cassette 1200. Air vent 906 pulls air into the metering pump 830 (shown in the mid-plate portion 1000 of blood pump cassette 1200 in FIG. 44). A second port 902 pushes air to the spike/source container, and also draws liquid from the source container 1202, which is then pushed by the metering pump 830 to the fluid line at point 826, where it enters the blood flow path of the blood pump cassette 1200. Valves 832, 834 and 836 are actuated in the proper order to ensure that the proper sequence of fluid withdrawal from source container 1202 and infusion into the blood path of the cassette occurs, followed by periodic air injection from air vent 906 into source container 1202 for purposes of pressure equalization within the container.

Blood output from the blood pump 13 (the outlet is located at a bottom of the pump 13) flows to an inlet of the dialyzer 14 (at the top of the dialyzer 14), and out of the dialyzer (the dialyzers blood outlet is located at the bottom of the dialyzer 14) to the inlet of the air trap 19. The outlet of the air trap 19 is connected to the venous blood line 204. Connections to the inlet and outlet blood ports of the dialyzer 14 are made with typical screw-type connections.

In other examples, the air trap 19 may be placed in the blood flow path after the blood exits the dialyzer and before it is returned to the patient. The air trap 19 can have a spherical or spheroid-shape container (i.e., a container having an approximately spherical inner wall), and have its inlet port located near the top and offset from the vertical axis of the container, and an outlet at a bottom of the container. (The vertical axis of the container is arranged in a vertical direction passing through the top and bottom "poles" of the approximately spherical container.) With the inlet port offset from the vertical axis (in this case set back toward the tray 171), blood is introduced into the container in a direction that is approximately perpendicular to the vertical axis of the container and that is approximately tangential to the spherical inner wall of the container. The curved shape of the inside wall of the trap can thus direct the blood to circulate along the inside wall as the blood gravitates to the bottom of the container (e.g., in a spiral like fashion), facilitating the removal of air bubbles from the blood. Air present in the blood exiting the outlet of the dialyzer 14 will enter at the top of the air trap 19 and remain at the top of the container as blood flows out the outlet at the bottom and to the venous blood line 204. By locating the inlet port near the top of trap 19, it is also possible to circulate blood through the trap with minimal or no air present within the container (as a "run-full" air trap. The ability to avoid an air-blood interface for routine circulation of blood in the trap can be advantageous. Placing the inlet port at or near the top of the container also allows most or all of the air present in the trap to be removed from the trap by reversing the flow of fluid through the blood tubing (i.e. from the bottom to the top of the trap 19, exiting through the inlet port of the trap 19).

A self-sealing port, such as a self-sealing stopper with a split septum or membrane, or another arrangement, may be located at the top of the trap, allowing the withdrawal of air from the container (e.g., by syringe). The blood-side surface of the self-sealing membrane can be situated nearly flush with the top of the interior of the trap, in order to facilitate cleaning of the self-sealing port during disinfection, e.g., by reversing flow through the air trap using a dialysate or other cleaning fluid. Also, the inlet, outlet and internal wall of the container and the self-sealing port may be arranged to substantially eliminate stagnation regions, i.e., allow for few or no regions where blood can stagnate or clot. The self-sealing port can also serve as a blood sampling site, and/or to allow the introduction of liquids, drugs or other compounds into the blood circuit. A sealed rubber-type stopper can be used if access with a needle is contemplated. Using a self-sealing stopper with split septum permits sampling and fluid delivery using a needleless system.

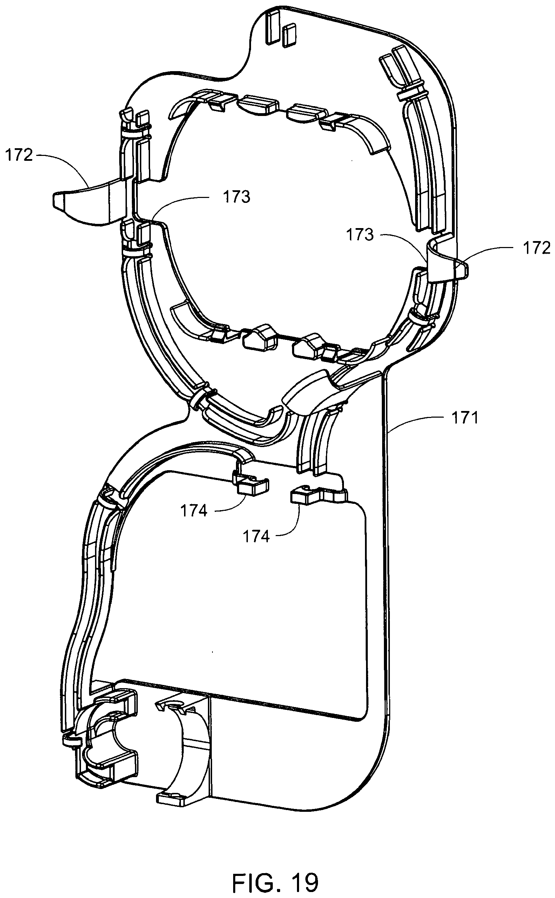

FIG. 19 shows the organizing tray 171 for the blood circuit assembly 17 without the various blood circuit assembly 17 components mounted. In accordance with the present invention, the organizing tray 171 includes handles 172 (in this embodiment, finger pulls) that a user can grip when mounting/dismounting the blood circuit assembly 17 to the front panel 511. Inward of the handles 172 are openings 173 that allow spring tabs on the front panel 511 to pass through and engage with the organizing tray 171 and/or the blood pump 13 cassette to hold the blood circuit assembly 17 in place on the front panel 511. In the organizing tray 171 includes blood line engagement members 174 that each have a C-shaped recess or other hole through which a corresponding blood line 203, 204 passes. (In this context, a "hole" includes a recess like that shown in FIG. 19, a throughbore that has a continuous wall, e.g., as may be made by a drill, or other suitable opening.) As described in more detail below, the blood line engagement members 174 are used when mounting the blood lines 203, 204 in the occluder 513. In short, when mounting the blood lines 203, 204 in the occluder 513, the blood lines 203, 204 must be pulled and stretched downwardly (so as to reduce the outside diameter of the line) while being pushed horizontally into slots for the occluder 513. The blood line engagement members 174 function to both resist downward pulling on the blood lines 203, 204 (e.g., each line 203, 204 may include a stop ring above the respective engagement member 174 that cannot be pulled through the recess in the engagement member 174) as well as permit the user to press inwardly on the engagement member 174 to seat the lines 203, 204 in the occluder slots. The engagement members 174 are formed integrally with the organizing tray 171 so that a "living hinge" or relatively flexible portion of the organizing tray is positioned between the engagement member 174 and the main body of the organizing tray 171. This arrangement allows the engagement members 174 to be pushed inwardly relative to the organizing tray 171 as the connection portion between the engagement members 174 and the organizing tray main body flexes.

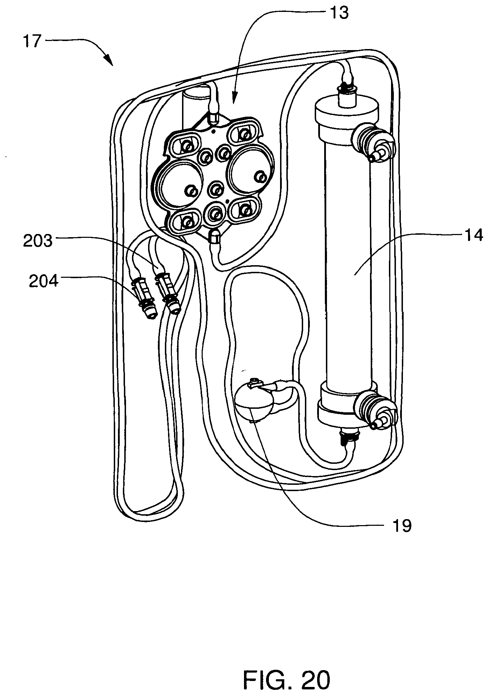

FIG. 20 shows a rear view of the blood circuit assembly 17 with the organizing tray 171 removed. This view shows the rear side of the blood pump 13 section with control ports exposed. These control ports mate with corresponding ports 515 on the front panel 511 (see FIG. 17) so that pneumatic control (e.g., suitable air pressure or vacuum) can be applied to the pumps and valves to control their operation and flow through the blood circuit assembly 17. FIG. 20 also shows the offset of the inlet port of the air trap 19, i.e., the inlet port at the top of the air trap 19 is arranged to the rear of the vertical axis of the generally spherical container portion of the air trap 19.

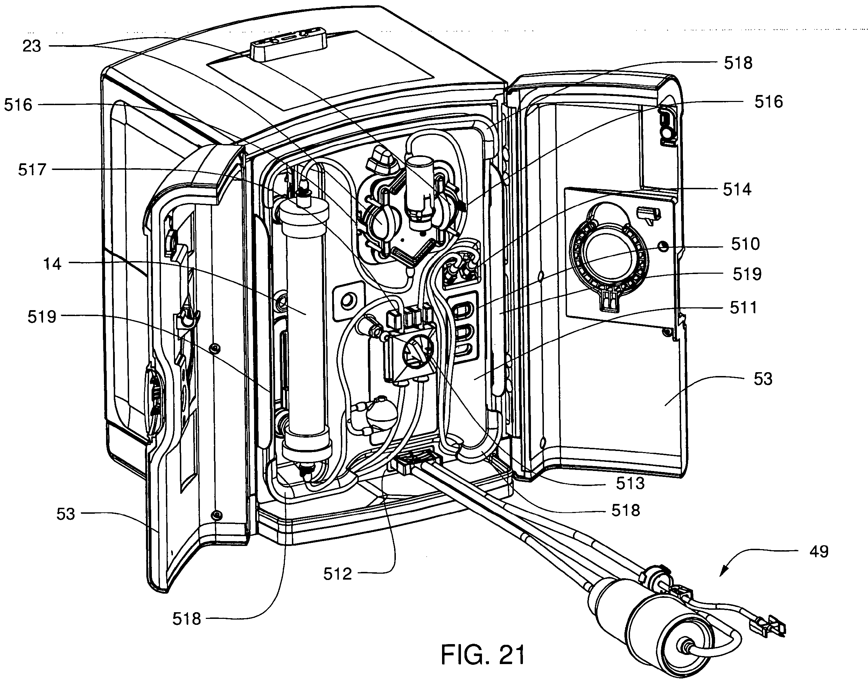

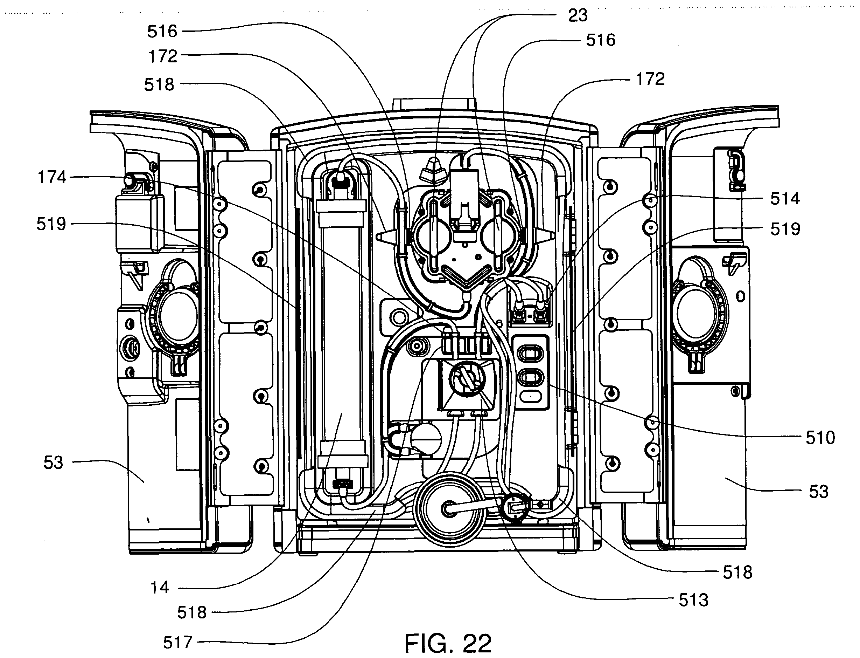

FIG. 21 shows a perspective view of the front panel 511 of the dialysis unit 51 with the blood circuit assembly 17 according to the present invention mounted to the front panel 511 without the organizing tray 171. (Normally, the blood circuit assembly 17 would include the organizing tray 171, but the tray 171 is not shown in the example so as to more clearly show components at the front panel 511.) On opposite sides of the blood pump 13 cassette, the front panel 511 has spring tabs 516 that extend forwardly and resiliently engage with the blood pump cassette and/or the organizing tray 171 to retain the blood circuit assembly 17 in place. The tabs 516 may include a barb or other feature to help retain the blood circuit assembly 17 in place. The spring tabs 516 may be flexed outwardly to release their hold on the blood circuit assembly 17, allowing its removal. However, in the absence of an outwardly directed force on the spring tabs 516, the tabs 516 will remain engaged with the blood circuit assembly 17. FIG. 22 shows a front view of the front panel 511 with the organizing tray 171 of the blood circuit assembly 17 included. To remove the blood circuit assembly 17 from the front panel 511, a user may place index fingers behind the handles 172 while simultaneously placing thumbs on the inner side of the spring tabs 516 (the sides nearest the blood pumps 23) and flexing the spring tabs 516 outwardly and away from the pumps 23. This causes the spring tabs 516 to release the blood circuit, assembly 17, e.g., disengagement of barbs on the tabs 516 from the blood pump 13 and/or the organizing tray 171. Of course, to remove the blood circuit assembly 17, other connections must be removed, including connections to the dialyzers 14 and the blood line connections points 514, as well as removal of the lines 203, 204 from the occluder 513. When mounting the blood circuit assembly 17 to the front panel 511, the organizing tray 171 may be grasped at the handles 172 and properly aligned, e.g., so that the spring tabs 516 are aligned to pass through the openings 173 and the control ports of the blood pump 13 cassette are aligned with the corresponding ports 515 on the front panel 511. The blood circuit assembly 17 may then be simply pushed into place, so that the spring tabs 516 engage with the organizing tray 171 and/or the blood pump cassette. Other connections can then be made, such as connections to the dialyzer 14, mounting of the blood lines 203,204 with the occluder 513, etc.

FIG. 21 also shows the slots 517 that hold the blood lines 203, 204 for leading into the occluder 513. The slots 517 define a channel that is slightly smaller than the outside diameter of the blood lines 203, 204 so that the lines 203, 204 tend to remain in the slots 517 after placement in the slots. This helps to ensure proper association of the lines with the occluder 513. Once the blood circuit assembly 17 is mounted on the spring tabs 516, the user may then engage the blood lines 203, 204 with the slots 517 by stretching the lines 203, 204 downward (with the engagement members 174 on the organizing tray 171 engaging the stop ring or other feature on the respective line 203, 204 and resisting the downward pull) and pushing the lines 203, 204 into a corresponding slot. The lines 203, 204 can be pushed into place by pressing inwardly on the engagement members 174, which as described above, are flexible and bend inwardly relative to the organizing tray 171. The lines 203, 204 can then be routed through the occluder 513.

The front panel 511 may include a blood line wrap feature around the periphery of the front panel 511. In this illustrative example, the front panel 511 includes flanged portions 518 along the top edge and at lower corners of the front panel 511. This allows a user to wrap the blood lines 203, 204 around the periphery of the front panel 511 by placing the lines 203, 204 in a channel defined by the flanged portions 518. The lines 203, 204 may be wrapped in a clockwise direction, starting from a point near the bottom of the dialyzer 14, and ending at a point near the lower right corner of the front panel 511. The blood lines 203, 204 may then be connected at the blood line connection points 514, e.g., to allow disinfecting fluid to be circulated through the blood lines 203, 204. As a result, the blood lines 203, 204 can be neatly retained on the front panel 511, allowing easy access to other components on the front panel 511 and allowing the user to close the doors 53 with minimal concern for pinching the blood lines 203, 204 between the doors 53 and the dialyzer unit housing 51. Alternatively, the blood lines 203, 204 may be first connected at the blood line connection points 514, and then wrapped in a clockwise direction, starting from a point near the bottom of the dialyzer 14, and ending at a point near the lower right corner of the front panel 511. This ensures that the blood lines are properly distributed along the flanged portions 518 to reach the connection points 514. Vertical fences 519 may also be provided along the left and right sides of the front panel 511 to help keep the blood lines 203, 204 in a desired position and away from the hinge plates 533 and other possible pinch points.

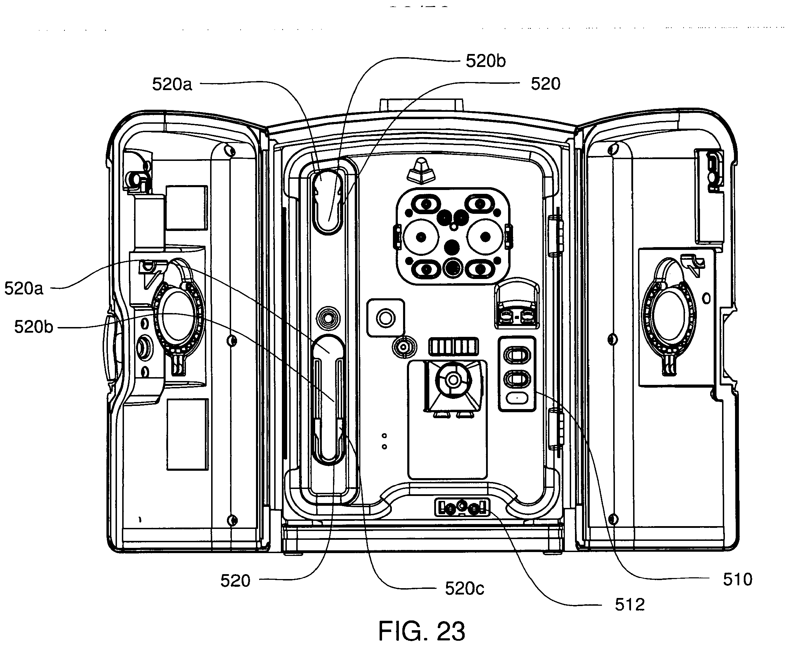

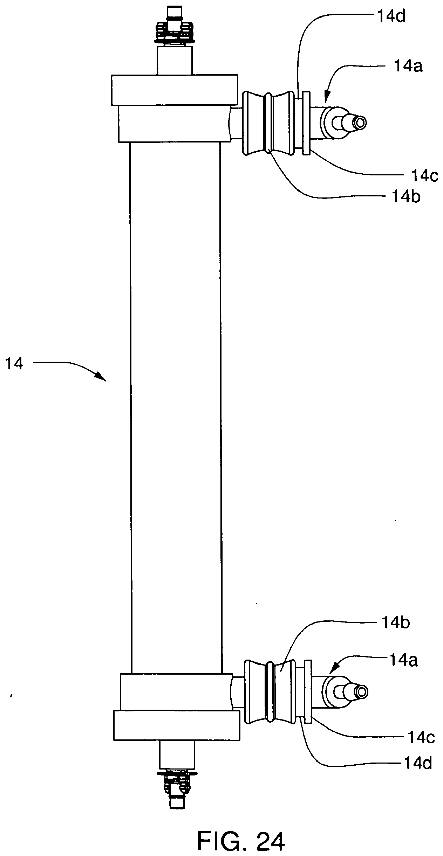

The front panel 511 of the dialysis unit 51 (or other suitable component) may be arranged to accommodate a variety of differently sized and/or shaped dialyzer units 14. Different patients, and in some cases even the same patient over time, may be prescribed different dialyzers so as to provide different treatment conditions. Thus, the dialysis unit 51 is preferably arranged to operate with multiple different types of dialyzers 14. In many cases, different dialyzers 14 have different dimensions, such as the overall diameter and/or length of the dialyzer unit. In this illustrative example as shown in FIG. 23, the front panel 511 includes a dialyzer mount with a pair of "keyhole" features 520 that are arranged to engage with a respective dialysate quick-connect fitting on the dialyzer 14. Each keyhole feature 520 includes an upper insertion area 520a sized to receive a portion of the quick-connect fitting and a lower flanged portion 520b that has a width that is smaller than an overall diameter of the quick-connect fitting and that engages with a grooved area of the quick-connect fitting. So as to aid in understanding of these features, FIG. 24 shows a dialyzer 14 with quick connect fittings 14a attached at dialysate inlet and outlet ports of the dialyzer 14. (Blood inlet and outlet ports are located at the extreme top and bottom of the dialyzer 14 shown in FIG. 24.) The quick connect fittings 14a shown are of a standard type, and most, if not all, dialyzers 14 have dialysate inlet/outlet ports that are arranged to engage with the standard quick connect fittings 14a. The quick connect fittings 14a each include a slide element 14b that is moved to the right (as shown in FIG. 24) relative to a base 14c to allow the fitting 14a to be engaged with a dialysate port on the dialyzer 14. When the slide element 14b is moved to allow the fitting 14a to be attached to the dialyzer 14, a groove 14d is closed. However, once the fitting 14a is properly seated on the inlet/outlet port of the dialyzer 14, the slide element 14b may be released, allowing a spring (not shown) to move the slide to the left as shown in FIG. 24, reestablishing the groove 14d to the condition shown in FIG. 24. Thus, when the quick connect fitting 14a is properly engaged with the dialyzer 14, the groove 14d will be present as shown in FIG. 24.

To mount the dialyzer 14 to the keyhole features 520, the quick connect fittings 14a may be partially inserted into the upper insertion area 520a of the top and bottom keyhole features, respectively, so that the groove 14d of each fitting 14a is aligned with a flange of the lower flanged portion 520b of the keyhole features 520. (Note that the upper insertion area 520 of the bottom keyhole feature 520 may be made longer than that shown in FIG. 23 to allow the accommodation of a wider range of dialyzer lengths.) With the grooves 14d aligned with the flanges, the dialyzer 14 may be lowered so that the quick connect fittings 14a are fully received into the lower flanged portions 520b of the keyhole features 520.

One or both of the keyhole features 520 may be adjustable so that the weight of the dialyzer 14 is shared by both lower flanged portions 520b of the keyhole features 520. For example, in this illustrative example, the bottom keyhole feature 520 has part of the lower flanged portion 520b adjustable in vertical position relative to the top keyhole feature 520. In this way, the portion of the lower flanged portion 520b may be adjusted in vertical position so that, with the top quick connect fitting 14a supported by the flanged portion 520b of the top keyhole feature 520, the movable portion of the flanged portion 520b of the bottom keyhole feature can be moved, e.g., upwardly, so that the bottom quick connect fitting 14a is also supported by the flanged portion 520b. Thus, the weight of the dialyzer 14 can be shared by both keyhole features 520. The flanged portion 520b may be made adjustable in any suitable way. In this embodiment, the flanged portion 520b has a "U" shaped member 520c that is vertically slidable along the vertical flanges and can be fixed in place by tightening a set of thumb screws. The "U" shaped member 520c may engage the quick connect fitting 14a so that the "U" shaped member 520c supports the weight (at least in part) of the dialyzer 14.

Although in the example above, the dialyzer 14 is supported by keyhole features in the front panel 511, a support arrangement for the dialyzer may be configured in other ways. For example, the upper insertion area 520a is not necessarily required. Instead, only flange portions (e.g., in the shape of a "U" shaped flange having opposed flange portions) may be provided to engage the dialyzer quick connect fittings. The flange portions may be offset from the front surface of the front panel 511 to provide clearance for the fitting and allow the flange portions to engage with the grooves of the quick connect fittings. Also, the flange portions need not be provided in a vertical orientation as shown, but instead may be oriented at an angle to the vertical, e.g., in a horizontal arrangement. The flange portions may have a detent, catch, or other feature to help maintain the dialyzer in place as well.

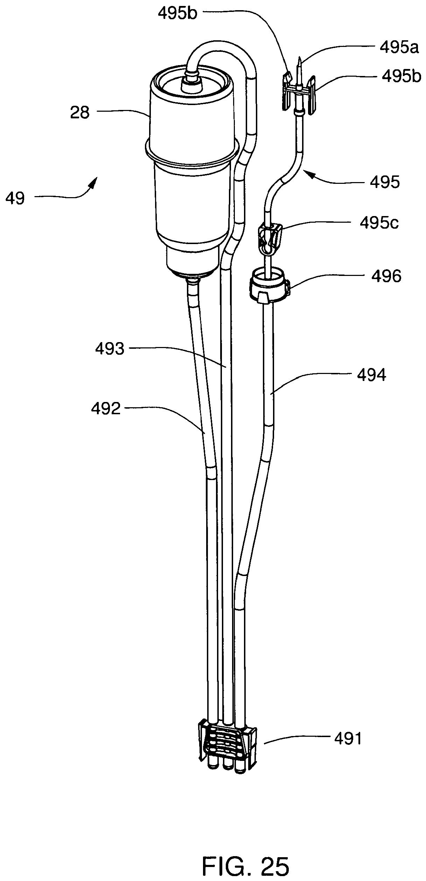

A bicarbonate, acid and/or other reagent supply device may be selectively associated with the dialysis unit. As described above, the dialysis unit 51 requires a supply of certain chemicals to generate dialysate and/or other materials needed for system operation. FIG. 25 shows a reagent supply 49 used to provide acid, bicarbonate and/or other materials to the dialysis unit 52. (FIG. 21 shows the reagent supply 49 attached to the acid/bicarbonate connection point 512 on the front panel 511.) The reagent supply 49 in this illustrative example includes an E-prong connector 491 that is arranged to mate with the acid/bicarbonate connection point 512. As with other connections made by the user at the front panel 511, e.g., including the blood line connections at the connection point 514, the mating connectors may be color coded or otherwise marked to help ensure proper connections are made. For example, the E-prong connector 491 and the acid/bicarbonate connection point 512 may be colored orange, while the arterial line 203 and its mating connection at the connection point 514 maybe colored red, and the venous line 204 and its mating connection at the connection point 514 are colored blue. Leading from the E-prong connector 491 are a bicarbonate supply line 492, a water supply line 493 and an acid supply line 494. (See FIG. 6 and the accompanying description regarding the function of these lines.) The water supply line 493 provides water to a bicarbonate supply 28 (which in this embodiment is a 750g Altracart Bicarbonate cartridge (#500750A) sold by Baxter International Inc. that includes a powdered bicarbonate material, but may be any suitable supply), which provides bicarbonate to the dialysis unit 51 via the bicarbonate supply line 492. In this example, the acid supply line 494 leads to an acid bag spike 495, which may be used to pierce and draw a suitable acid from a IV-type bag or other container. In this example, the acid bag spike 495 includes a spike member 495a and a pair of spring clips 495b. The spring clips 495b are joined together at center portions by a connecting bar such that the spring clips 495b and the connecting bar form an "H" shape and allow the spring clips 495b to be pivoted relative to each other when proximal ends of the spring clips 495b are squeezed toward each other. The spring clips 495b may be arranged to engage with a connector element on an acid bag (or other acid supply, not shown) so that the spike member 495a remains engaged with the bag until a user disengages the clips 495b. For example, distal ends of the clips 495b may include barbs that engage with the acid supply, and the clips may be disengaged from the acid supply by squeezing proximal ends of the clips 495b together to disengage the barb elements at the distal ends of the clips 495b from the acid supply. The acid bag spike 495 may also include a valve 495c (in this case, a pinch clamp) to open/close the line of the acid bag spike 495. In other examples, the acid bag spike 495 may be replaceable (disconnected from the acid supply line 494 at a cap connector 496) with another component, such as an acid jug straw (not shown) or other arrangement. When used with a jug straw, the cap connector 496 may be engaged with an acid jug opening such that the cap connector 496 covers the opening, like a cap. Alternatively, the jug straw can terminate in a spike, which then has the ability to penetrate a self-sealing (e.g. rubber) membrane covering the opening of the acid jug. Thus, different types of components may be attached to the acid supply line 494 depending on the acid supply arrangement (such as a jug, bottle, bag, or other).

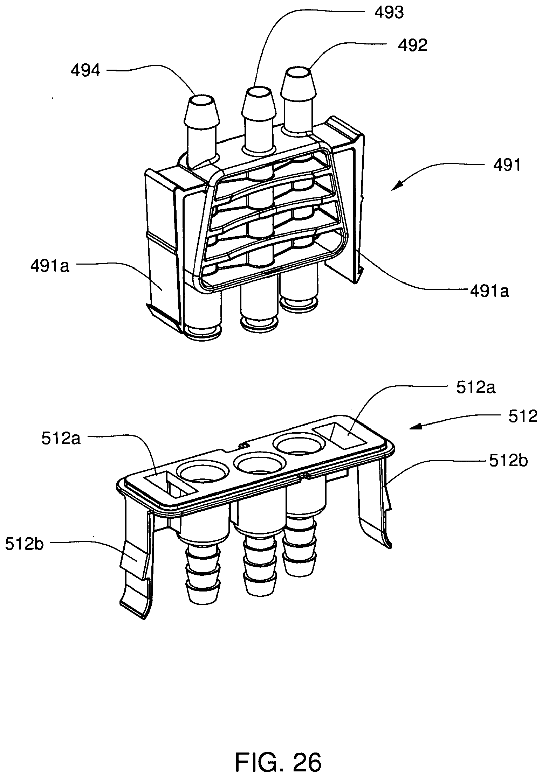

FIG. 26 shows a close up view of the E-prong connector 491 and the corresponding connection point 512 at the front panel 511. The E-prong connector 491 has three parallel prongs (corresponding to the bicarbonate and acid supply lines 492 and 494 and the water supply line 493) that that engage with corresponding receiving holes in the connection point 512. The E-prong connector 491 and receiving holes m the connection point 512 are arranged so that a center lumen (the water supply line 493) is arranged above, or otherwise out of, a common plane of the two outer lumens (the bicarbonate and acid supply lines 492 and 494). In this way, it is ensured that the bicarbonate and acid supply lines 492 and 494 are properly connected since the E-prong connector 491 cannot be engaged with the connection point 512 unless appropriately oriented. The E-prong connector 491 includes a pair of spring tabs 491a that can be engaged with corresponding slots 512a in the connection point 512, e.g., when the prongs are properly seated in receiving holes of the connection point 512. With the tabs 491a engaged in the slots 512a, the E-prong connector 491 cannot be easily removed from the connection point 512, helping reduce the likelihood of an accidental disconnection. The E-prong connector 491 may be disconnected by pressing the tabs 491a toward each other so that barbs at the distal ends of the tabs 491a disengage from the slots 512a. The connection point 512 has similar spring tabs 512b which allow the connection point 512 to be connected to and disconnected from the front panel 511.

A disinfect connector (not shown) may engage with connection point 512 for use during a disinfection procedure. The disinfect connector has three parallel prongs having a similar orientation as the E-prong connector 491, so that the prongs may engage with the receiving holes in connection point 512. The channels in the prongs of the disinfect connector terminate within a common chamber within the disinfect connector. Thus, during a disinfect procedure, the bicarbonate flow line, acid flow line and water flow line are all interconnected, permitting disinfection of each of these flow lines during the disinfect procedure. (This is shown as a dashed inverted "T" line at 49 in Fig. 6).

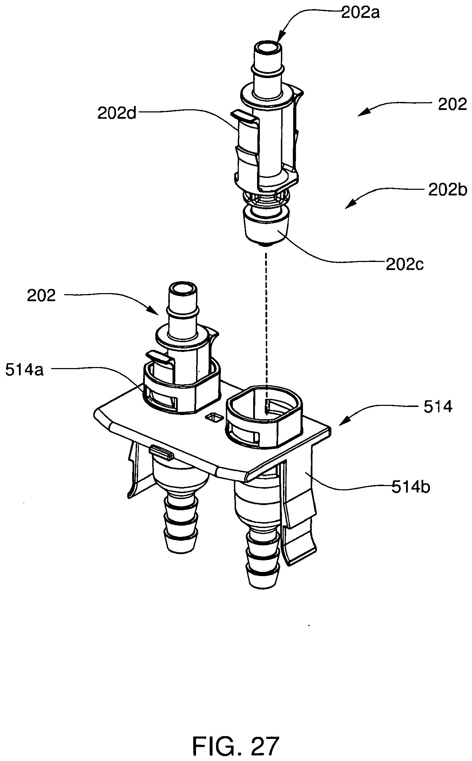

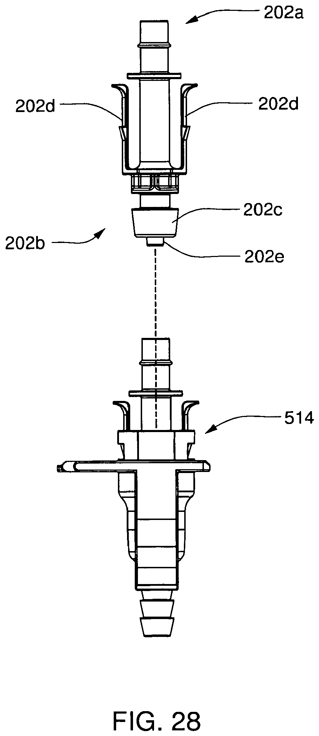

The blood lines 203, 204 may be equipped with a connector that enables two types of connections to be made. One type of connection is a plug-in or press-in connection by which the connector can be pushed into a receiving lumen and a leakfree connection made without requiring rotation of the connector or the receiving lumen. A second type of connection is a screw-type connection by which a leakfree connection can be made by a threaded engagement of the connector with a complementary element. For example, FIGs. 27 and 28 show a perspective view and a side view of a blood line connector 202 that is used with the blood lines 203, 204 and that can engage with the blood line connection point 514 on the front panel 511. The connector 202 includes a tube connection end 202a that connects to the corresponding blood line 203, 204, and a patient access connection end 202b that is arranged to connect to both a patient access as well as the connection point 514 to establish a leakfree connection. At the patient access connection end 202b, the connector 202 includes a frustoconical member 202c that has an internally threaded portion arranged to engage with an externally threaded patient access. For example, the frustoconical member 202c may be part of a male-type luer connector that includes the central tube 202e extending from the center of the frustoconical member 202c. When making the luer connection, the tube 202e may extend into a female luer connector at the patient access and the threaded portion on the interior of the frustoconical member 202c may engage with a thread on the female luer connector of the patient access (whether arterial or venous). Such luer connections are standard when connecting blood lines to a patient access. However, the connector 202 may also be engaged with the connection point 514 by simply pushing the patient access connection end 202b into a receiving hole of the connection point 514. When making this connection, the exterior of the frustoconical member 202c may engage with a suitable seat, or other surface or element in the connection point 514 (such as a valve seat, O-ring, or other) so that a seal is formed between the frustoconical member 202c and the connection point 514. The central tube 202e may also, or instead, be used to engage with the connection point 514 to establish a suitable seal. Locking arms 202d that extend rearwardly from the frustoconical member 202c may engage with holes 514a in the connection point 514 (e.g., barbed portions on the arms 202d may engage with the holes 514a) to help maintain the connector 202 in the receiving hole of the connection point 514. The connector 202 may be released by pressing the arms 202d toward each other (e.g., by pressing on finger depression portions at the distal ends of the arms 202d), thereby disengaging the barbs from the holes 514a, and withdrawing the connector 202. Note that the connection point 514 may include spring tabs 514b to allow the connection point 514 to be selectively engaged/disengaged at the front panel 511. The connectors 202 may be made in any suitable way, such as by molding of plastic as a single unitary part.

While several embodiments of the present invention and related examples have been described and illustrated herein, those of ordinary skill in the art will readily envision a variety of other means and/or structures for performing the functions and/or obtaining the results and/or one or more of the advantages described herein, within the scope of the claims which define the present invention. More generally, those skilled in the art will readily appreciate that all parameters, dimensions, materials, and configurations described herein are meant to be exemplary and that the actual parameters, dimensions, materials, and/or configurations will depend upon the specific application or applications for which the present teachings are used. Those skilled in the art will recognize, or be able to ascertain using no more than routine experimentation, many equivalents to the specific embodiments of the invention described herein, within the scope of the claims below. It is, therefore, to be understood that the foregoing embodiments are presented by way of example only and that, within the scope of the appended claims, the invention may be practiced otherwise than as specifically described.

The indefinite articles "a" and "an," as used herein in the specification and in the claims, unless clearly indicated to the contrary, should be understood to mean "at least one."

**Claims**

1. A blood circuit assembly for a dialysis unit, comprising:

    an organizing tray (171) having a pair of handles (172) arranged for gripping by a user for mounting or dismounting the blood circuit assembly on a front panel of the dialysis unit;
    a pair of pneumatic pumps (23) mounted to the organizing tray between the pair of handles for circulating blood received from a patient through a circuit including a dialyzer (14) and back to the patient;
    an air trap (19) mounted to the organizing tray arranged to remove air from blood circulating in the circuit;
    a pair of dialyzer connections arranged to connect to the inlet and outlet of a dialyzer; and
    a pair of blood line connectors (202), one inlet blood line connector for receiving blood from the patient and providing blood to the pneumatic pumps and the other outlet blood line connector for returning blood to the patient.

2. The blood circuit assembly of claim 1, further comprising an anticoagulant connection (1206) for engaging with an anticoagulant source (11, 1202) and providing anticoagulant into the circuit.

3. The blood circuit assembly of claim 2, further comprising a pump (80, 830) for pumping anticoagulant from the anticoagulant source to the circuit.

4. The blood circuit assembly of claim 3, wherein the pair of pneumatic pumps, the anticoagulant connection, and the anticoagulant pump are part of a pump cassette (13, 1200).

5. The blood circuit assembly of claim 4, wherein the inlet blood line connector is connected to an inlet for the pump cassette, an outlet for the pump cassette is connected to a dialyzer inlet connector, a dialyzer outlet connector is connected to an inlet of the air trap, and an outlet of the air trap is connected to the outlet blood line connector.

6. The blood circuit assembly of any preceding claim, wherein the pneumatic pumps include ports arranged to mate with corresponding control ports on the front panel for control of the pneumatic pumps.

7. The blood circuit assembly of any preceding claim, wherein the organizing tray includes openings (173) adjacent each of the handles for receiving retaining tabs (516) on a dialysis unit that engage with the blood circuit assembly and retain the blood circuit assembly on the dialysis unit.

8. The blood circuit assembly of any preceding claim, wherein the inlet of the air trap is located above the outlet of the air trap when the blood circuit assembly is mounted to a dialysis unit.

9. The blood circuit assembly of any preceding claim, wherein the pneumatic pumps are diaphragm pumps.

10. The blood circuit assembly of any preceding claim, wherein the blood line connectors (202) are arranged for a threaded luer-type connection to a patient access, and are arranged for a press-in type connection to the dialysis unit.

11. The blood circuit assembly of claim 10, wherein the blood line connectors include a frustoconical member (202c) with an internal thread for the luer-type connection, and a central tube (202e) extending from a center of the frustoconical member.

12. The blood circuit assembly of claim 11, wherein the blood line connectors include locking arms (202d) with at least one barb to engage with a complementary connector for the press-in type connection to the dialysis unit.

13. The blood circuit assembly of any preceding claim, wherein the organizing tray includes circuit tube engagement members (174) having a hole through which a respective circuit tube passes, the engagement members engaging with the respective circuit tube to allow the circuit tube to be pulled and stretched for engagement with an occluder of the dialysis unit.

14. A method for replacing a blood circuit assembly according to claim 1 of a dialysis unit, the method including:

    grasping the pair of handles (172) of the organizing tray (171) that is mounted to a dialysis unit (51),
    disengaging locking tabs (516) of the dialysis unit from the blood circuit assembly to free the blood circuit assembly from the dialysis unit; and
    pulling on the handles on the organizing tray of the blood circuit assembly to remove the blood circuit assembly from the dialysis unit.

15. The method of claim 14, wherein disengaging the locking tabs (516) is performed by flexing the locking tabs away from each other such that each locking tab is moved towards a nearest one of the handles (172).

16. The method of claim 14 or 15, further including:

after removal of the blood circuit assembly which is being replaced, providing a replacement blood circuit assembly;

aligning openings in the organizing tray (171) with the locking tabs (516) of the dialysis unit (51) so that each locking tab is received into a respective opening; and

pushing the organizing tray relative to the dialysis unit such that the locking tabs (516) engage with the replacement blood circuit assembly to mount the replacement blood circuit assembly to the dialysis unit.

17. The method of claim 16, wherein to mount the replacement blood circuit assembly to the dialysis unit further includes connecting control ports on the dialysis unit to mating ports on the blood circuit assembly so that fluid control signals may be provided for pumps and valves of the blood circuit assembly.

18. The method of any one of claims 14 to 17 further comprising making other blood circuit connections in the form of inlet and outlet connections for a dialyzer (14).

19. The method of any one of claims 14 to 18 further comprising making other blood circuit connections by connecting blood line connectors to receive dialysate into the blood circuit.

**Patentansprüche**

1. Blutkreislaufaufbau für eine Dialyseeinheit, die aufweist:

ein Organisationstablett (171), aufweisend ein Paar Griffe (172), das zum Greifen durch einen Benutzer zum Installieren oder Deinstallieren des Blutkreislaufaufbaus an einem Frontpaneel der Dialyseeinheit eingerichtet ist;
ein Paar von pneumatischen Pumpen (23), das an dem Organisationstablett zwischen dem Paar Griffe zum Umwälzen von von einem Patienten empfangenen Blut durch einen Kreislauf, der eine Dialyseeinrichtung (14) aufweist, und zurück zu dem Patienten montiert sind;
eine Luftfalle (19), die an dem Organisationstablett montiert ist und eingerichtet ist, um Luft von in dem Kreislauf zirkulierendem Blut zu entfernen;
ein Paar Dialyseverbindungen, die eingerichtet sind, um den Einlass und den Auslass einer Dialyseeinrichtung zu verbinden; und
ein Paar Blutleitungsverbinder (202), bei dem ein Bluteinlassleitungsverbinder zum Empfangen von Blut von dem Patienten und zum Bereitstellen von Blut an den pneumatischen Pumpen und der andere Blutauslassleitungsverbinder zum Rückführen von Blut zu dem Patienten ist.

2. Blutkreislaufaufbau nach Anspruch 1, ferner umfassend eine Antikoagulansverbindung (1206) für einen Eingriff mit einer Antikoagulansquelle (11, 1202) und eine Bereitstellung eines Antikoagulans in dem Kreislauf.

3. Blutkreislaufaufbau nach Anspruch 2, ferner umfassend eine Pumpe (80, 830) zum Pumpen des Antikoagulans von der Antikoagulansquelle zu dem Kreislauf.

4. Blutkreislaufaufbau nach Anspruch 3, bei dem das Paar pneumatischer Pumpen, die Antikoagulansverbindung und die Antikoagulanspumpe Teil einer Pumpkassette (13, 1200) sind.

5. Blutkreislaufaufbau nach Anspruch 4, bei dem der Bluteinlassleitungsverbinder mit einem Einlass für die Pumpkassette verbunden ist, ein Auslass für die Pumpkassette mit einem Dialyseeinrichtungseinlassverbinder verbunden ist, ein Dialyseeinrichtungsauslassverbinder mit einem Einlass der Luftfalle verbunden ist und ein Auslass der Luftfalle mit dem Blutauslassleitungsverbinder verbunden ist.

6. Blutkreislaufaufbau nach einem der vorstehenden Ansprüche, bei dem die pneumatischen Pumpen Anschlüsse aufweisen, die eingerichtet sind, um sich mit entsprechenden Steueranschlüssen an dem Frontpaneel zum Steuern der pneumatischen Pumpen zu verbinden.

7. Blutkreislaufaufbau nach einem der vorstehenden Ansprüche, bei dem das Organisationstablett Öffnungen (173) aufweist, die benachbart zu jedem der Griffe zum Aufnehmen von Haltelaschen (516) an einer Dialyseeinheit sind, die mit dem Blutkreislaufaufbau in Eingriff gehen und den Blutkreislaufaufbau an der Dialyseeinheit halten.

8. Blutkreislaufaufbau nach einem der vorstehenden Ansprüche, bei dem der Einlass der Luftfalle über dem Auslass der Luftfalle angeordnet ist, wenn der Blutkreislaufaufbau an einer Dialyseeinheit montiert ist.

9. Blutkreislaufaufbau nach einem der vorstehenden Ansprüche, bei dem die pneumatischen Pumpen Membranpumpen sind.

10. Blutkreislaufaufbau nach einem der vorstehenden Ansprüche, bei dem die Blutleitungsverbinder (202) für eine Luer-Gewindeverbindung mit einem Patientenzugang eingerichtet sind und für eine Eindrückverbindung mit der Dialyseeinheit eingerichtet sind.

11. Blutkreislaufaufbau nach Anspruch 10, bei dem die Blutleitungsverbinder ein Kegelstumpfelement (202c) mit einem Innengewinde für die Luerverbindung und ein mittiges Rohr (202e) aufweisen, das sich von einer Mitte des Kegelstumpfelements erstreckt.

12. Blutkreislaufaufbau nach Anspruch 11, bei dem die Blutleitungsverbinder Verriegelungsarme (202d) mit mindestens einem Haken aufweisen, um mit einem komplementären Verbinder für die Eindrückverbindung zu der Dialyseeinheit in Eingriff zu gehen.

13. Blutkreislaufaufbau nach einem der vorstehenden Ansprüche, bei dem das Organisationstablett Kreislaufrohreingriffselemente (174) aufweist, die ein Loch aufweisen, durch das ein jeweiliges Kreislaufrohr hindurchgeht, wobei die Eingriffselemente mit einem jeweiligen Kreislaufrohr in Eingriff sind, um es den Kreislaufrohren zu ermöglichen, für einen Eingriff mit einem Occluder der Dialyseeinheit gezogen und gedehnt zu werden.

14. Verfahren zum Ersetzen eines Blutkreislaufaufbaus nach Anspruch 1 einer Dialyseeinheit, wobei das Verfahren umfasst:

   Greifen des Paars Griffe (172) des Organisationstabletts (171), das an einer Dialyseeinheit (51) befestigt ist,
   Lösen der Haltelaschen (516) der Dialyseeinheit von dem Blutkreislaufaufbau zum Freisetzen des Blutkreislaufaufbaus von der Dialyseeinheit; und
   Ziehen an den Griffen an dem Organisationstablett des Blutkreislaufaufbaus zum Entfernen des Blutkreislaufaufbaus von der Dialyseeinheit.

15. Verfahren nach Anspruch 14, wobei Lösen der Haltelaschen (516) durch Biegen der Haltelaschen voneinander weg erfolgt, sodass jede Haltelasche in Richtung zu einem nächsten der Griffe (172) bewegt wird.

16. Verfahren nach Anspruch 14 oder 15, weiterhin umfassend:

   nach Entfernen des Blutkreislaufaufbaus, der ersetzt wird, Bereitstellen eines Ersatz-Blutkreislaufaufbaus;
   Ausrichten der Öffnungen in dem Organisationstablett (171) mit den Haltelaschen (516) der Dialyseeinheit (51), sodass jede Haltelaschen in einer jeweiligen Öffnung aufgenommen wird; und

   Schieben des Organisationstabletts relativ zu der Dialyseeinheit, sodass die Haltelaschen (516) mit dem Ersatz-Blutkreislaufaufbau in Eingriff kommen, um den Ersatz-Blutkreislaufaufbau an der Dialyseeinheit zu befestigen.

17. Verfahren nach Anspruch 16, wobei das Befestigen des Ersatz-Blutkreislaufaufbaus an der Dialyseeinheit weiterhin Anschließen von Steueranschlüssen an der Dialyseeinheit zum Zusammenfügen mit Anschlüssen an dem Blutkreislaufaufbau einschließt, sodass Fluid-Steuer-Signale für Pumpen und Ventile der Blutkreislaufanordnung bereitgestellt werden können.

18. Verfahren nach einem der Ansprüche 14 bis 17, ferner umfassend Schaffen anderer Blutkreislauf-Verbindungen in Form von Einlass- und Auslass-Verbindungen für eine Dialyseeinrichtung (14).

19. Verfahren nach einem der Ansprüche 14 bis 18, ferner umfassend Schaffen anderer Blutkreislauf-Verbindungen durch Anschließen von Blut-Leitungs-Verbindungen zur Aufnahme von Dialysat in den Blutkreislauf.

**Revendications**

1. Ensemble de circuit sanguin pour une unité de dialyse, comprenant :

   un plateau organiseur (171) ayant une paire de poignées (172) aménagées pour être saisies par un utilisateur pour monter ou démonter l'ensemble de circuit sanguin sur un panneau avant de l'unité de dialyse ;
   une paire de pompes pneumatiques (23) montées sur le plateau organiseur entre la paire de poignées pour faire circuler du sang reçu d'un patient à travers un circuit comportant un dialyseur (14) et le renvoyer au patient ;
   un piège à air (19) monté sur le plateau organiseur et aménagé pour extraire l'air du sang circulant dans le circuit ;
   une paire de raccords de dialyseur aménagés pour se raccorder à l'entrée et à la sortie du dialyseur; et
   une paire de raccords de conduit sanguin (202), un raccord d'entrée de conduit sanguin pour recevoir du sang du patient et fournir du sang aux pompes pneumatiques et l'autre raccord de sortie de conduit sanguin pour renvoyer le sang au patient.

2. Ensemble de circuit sanguin selon la revendication 1, comprenant en outre un raccord d'anticoagulant (1206) pour s'engager avec une source d'anticoagulant (11, 1202) et fournir de l'anticoagulant dans le circuit.

3. Ensemble de circuit sanguin selon la revendication 2, comprenant en outre une pompe (80, 830) destinée à pomper de l'anticoagulant de la source d'anticoagulant dans le circuit.

4. Ensemble de circuit sanguin selon la revendication 3, dans lequel la paire de pompes pneumatiques, le raccord d'anticoagulant, et la pompe d'anticoagulant font partie d'une cassette de pompe (13, 1200).

5. Ensemble de circuit sanguin selon la revendication 4, dans lequel le raccord d'entrée de conduit sanguin est raccordé à une entrée de la cassette de pompe, une sortie de la cassette de pompe est raccordée à un raccord d'entrée de dialyseur, un raccord de sortie de dialyseur est raccordé à une entrée du piège à air, et une sortie du piège à air est raccordée au raccord de sortie de conduit sanguin.

6. Ensemble de circuit sanguin selon une quelconque revendication précédente, dans lequel les pompes pneumatiques comportent des orifices aménagés pour s'apparier à des orifices de commande correspondants du panneau avant pour une commande des pompes pneumatiques.

7. Ensemble de circuit sanguin selon une quelconque revendication précédente, dans lequel le plateau organiseur comporte des ouvertures (173) adjacentes à chacune des poignées et destinées à recevoir des pattes de retenue (516) sur une unité de dialyse qui s'engagent avec l'ensemble de circuit sanguin et retiennent l'ensemble de circuit sanguin sur l'unité de dialyse.

8. Ensemble de circuit sanguin selon une quelconque revendication précédente, dans lequel l'entrée du piège à air se trouve au-dessus de la sortie du piège à air lorsque l'ensemble de circuit sanguin est monté sur une unité de dialyse.

9. Ensemble de circuit sanguin selon une quelconque revendication précédente, dans lequel les pompes pneumatiques sont des pompes à membrane.

10. Ensemble de circuit sanguin selon une quelconque revendication précédente, dans lequel les raccords de conduit sanguin (202) sont aménagés pour un raccordement de type Luer à vis avec un accès au patient, et sont aménagés pour un raccordement de type à enfoncement avec l'unité de dialyse.

11. Ensemble de circuit sanguin selon la revendication 10, dans lequel les raccords de conduit sanguin comportent un élément tronconique (202c) avec un filetage interne pour le raccordement de type Luer, et un tube central (202e) s'étendant du centre de l'élément tronconique.

12. Ensemble de circuit sanguin selon la revendication 11, dans lequel les raccords de conduit sanguin comportent des bras de verrouillage (202d) avec au moins un barbillon pour s'engager avec un raccord complémentaire pour le raccordement de type à enfoncement avec l'unité de dialyse.

13. Ensemble de circuit sanguin selon une quelconque revendication précédente, dans lequel le plateau organiseur comporte des éléments d'engagement de tubes de circuit (174) ayant un trou par lequel passe un tube de circuit respectif, les éléments d'engagement s'engageant avec les tubes de circuit respectifs pour permettre que les tubes de circuit soient tirés et allongés pour s'engager avec un obturateur de l'unité de dialyse.

14. Procédé de remplacement d'un ensemble de circuit sanguin selon la revendication 1 d'une unité de dialyse, le procédé comportant :

la saisie de la paire de poignées (172) du plateau organisateur (171) qui est monté sur une unité de dialyse (51),
le désengagement de pattes de verrouillage (516) de l'unité de dialyse de l'ensemble de circuit sanguin pour libérer l'ensemble de circuit sanguin de l'unité de dialyse ; et
la traction sur les poignées sur le plateau organisateur de l'ensemble de circuit sanguin pour retirer l'ensemble de circuit sanguin de l'unité de dialyse.

15. Procédé selon la revendication 14, dans lequel le désengagement des pattes de verrouillage (516) est effectué en fléchissant les pattes de verrouillage loin l'une de l'autre de sorte que chaque patte de verrouillage soit déplacée vers la plus proche des poignées (172).

16. Procédé selon la revendication 14 ou 15, comportant en outre :

après le retrait de l'ensemble de circuit sanguin qui est remplacé, la fourniture d'un ensemble de circuit sanguin de remplacement;
l'alignement d'ouvertures dans le plateau organisateur (171) avec les pattes de verrouillage (516) de l'unité de dialyse (51) de sorte que chaque patte de verrouillage soit reçue dans une ouverture respective ; et
la poussée du plateau organisateur par rapport à l'unité de dialyse de sorte que les pattes de verrouillage (516) s'engagent avec l'ensemble de circuit sanguin de remplacement pour monter l'ensemble de circuit sanguin de remplacement sur l'unité de dialyse.

17. Procédé selon la revendication 16, dans lequel le montage de l'ensemble de circuit sanguin de remplacement sur l'unité de dialyse comporte en outre le raccordement d'orifices de commande sur l'unité de dialyse à des orifices d'appariement sur l'ensemble de circuit sanguin de sorte que des signaux de commande de fluide puissent être fournis pour des pompes et valves de l'ensemble de circuit sanguin.

18. Procédé selon l'une quelconque des revendications 14 à 17 comprenant en outre la réalisation d'autres raccordements de circuit sanguin sous la forme de raccordements d'entrée et de sortie d'un dialyseur (14).

19. Procédé selon l'une quelconque des revendications 14 à 18 comprenant en outre la réalisation d'autres raccordements de circuit sanguin en raccordant des raccords de conduit sanguin pour recevoir un dialysat dans le circuit sanguin.

FIG.1

FIG. 2

FIG. 3

FIG. 4

FIG. 5

FIG. 6

FIG. 7

EP 2 197 513 B1

FIG. 8

FIG. 9

FIG. 10

FIG. 11

FIG. 12

FIG. 13

FIG. 14

FIG. 15

FIG. 16

EP 2 197 513 B1

FIG. 17

FIG. 18

FIG. 19

FIG. 20

FIG. 21

FIG. 22

FIG. 23

FIG. 24

EP 2 197 513 B1

FIG. 25

54

FIG. 26

FIG. 27

202a

202d — 202d

202b

202c

202e

514

FIG. 28

FIG. 29

EP 2 197 513 B1

FIG. 30

FIG. 31

FIG. 32A

FIG. 32B

FIG. 33A

FIG. 33B

4300

4310
4312
4316
4314

4320

4316
4314

4334
4332
4334

4330

4346
4346
4346
4346
4342
4346

4340

FIG. 34A

4300

4344

4310

4342

4312

4346

4334

4314

4318

FIG. 34B

4350

4352

FIG. 34C

4300

FIG. 35A

FIG. 35B

FIG. 36A

FIG. 36B

FIG. 37

EP 2 197 513 B1

STEP 1

INITIALIZE
SYSTEM

470

STEP 2

FILL PUMP
CHAMBER

• OPEN INLET VALVE

• MOVE PISTON
TO POSITION 3
DISPLACING ΔV

• CLOSE INLET
VALVE TO ISOLATE
PUMP CHAMBER

472

STEP 3

VOLUME CALCULATION
AND
GAS DETECTION
SUB CYCLE

(A)

474

(C)

STEP 4

DELIVER FLUID

• OPEN OUTLET VALVE

• MOVE PISTON
TO POSITION 1

• CLOSE OUTLET
VALVE TO ISOLATE
PUMP CHAMBER

208

STEP 5

VOLUME CALCULATION
AND VOLUME DELIVERED

OPTIONAL

210

REPEAT TO BEGIN
NEW PUMP CYCLE

FIG. 38A

(A)

MEASURE $P_1$ IN CONTROL CHAMBER — 474 — SUB-STEP 1

MOVE PISTON FROM 3 TO 1 REDUCING VOLUME OF MEASUREMENT GAS BY $\Delta V$ — 478 — SUB-STEP 2

MEASURE $P_2$ IN CONTROL CHAMBER — 480 — SUB-STEP 3

VOLUME CALCULATION

DETERMINE VOLUME OF PUMP CHAMBER VIA IDEAL GAS LAW AND CONSERVATION OF MASS: — 482 — SUB-STEP 4

$$V_{F_1} = V_T - \frac{P_2 \Delta V}{P_2 - P_1}$$

OPEN CONTROL CHAMBER VENT VALVE TO EQUILIBRATE CONTROL CHAMBER PRESSURE TO ATMOSPHERIC PRESSURE THEN CLOSE VENT VALVE — 484 — SUB-STEP 5

MEASURE NEW $P_1$ IN CONTROL CHAMBER — 486 — SUB-STEP 6

GAS DETECTION

MOVE PISTON FROM 1 TO 3 INCREASING VOLUME OF MEASUREMENT GAS BY $\Delta V$ — 488 — SUB-STEP 7

MEASURE NEW $P_2$ IN CONTROL CHAMBER — 490 — SUB-STEP 8

CALCULATE $V_{F_2}$ AS ABOVE FOR $V_{F_1}$ — 400 — SUB-STEP 9

COMPARE $[V_{F_1} - V_{F_2}]$ — 402 — SUB-STEP 10

(B)

## FIG. 38B

FIG. 38C

FIG. 39

FIG. 40

FIG. 41

1200

1202

1206

1204

FIG. 42

FIG. 43

1000

FIG. 44

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006120415 A
- US 20070253463 A1
- WO 2008053259 A1